# EUROPEAN PATENT APPLICATION

(11) **EP 4 309 598 A2**
(43) Date of publication of application: **24.01.2024**
(21) Application number: 23180036.8
(22) Date of filing: 19.06.2023
(51) Int. Cl.: A61B 17/34, A61B 5/145, A61B 5/1486, A61B 5/00, A61M 5/158

(54) **INTEGRATED SUBCUTANEOUS SENSOR AND INFUSION DEVICE, SYSTEM AND METHOD**

(30) Priority: 24.06.2022 US 202263355274 P; 19.04.2023 US 202318303496
(71) Applicant: MEDTRONIC MINIMED, INC., Northridge, CA 91325-1219 (US)
(72) Inventor: Chiu, Chia-Hung, Northridge, 91325-1219 (US); Zanabria, Elizabeth, Northridge, 91325-1219 (US); Gottlieb, Rebecca K., Culver City, 90230 (US); Das, Biswa Prakash, Irvine, 92620 (US); Garai, Ellis, Northridge, 91325-1219 (US); Kelleher, Kevin Michael, Northridge, 91325-1219 (US); Wang, Hsifu, 108 Taipei (TW); Namani, Ravi, Northridge, 91325-1219 (US)
(74) Representative: Zimmermann & Partner Patentanwälte mbB

(57) **Abstract**

A medical device includes a base having a first surface to be secured to a patient's skin. A first insertable member has a length portion extending from the first surface of the base for insertion. A second insertable member has a length portion extending from the first surface of the base for insertion through the patient's skin. The first insertable member includes a sensor member for sensing a biological condition, and the second insertable member includes an infusion cannula for infusing an infusion media. The distal end of the first insertable member and the distal end of the second insertable member are spaced apart by a first distance of at least 5.0 mm, for reducing interference of the infusion media from the infusion cannula with an operation of the sensor member.

## Description

### CROSS REFERENCE TO RELATED APPLICATION

This application claims priority to U.S. Provisional Application No. 63/355,274, filed June 24, 2022, entitled "INTEGRATED SUBCUTANEOUS SENSOR AND INFUSION DEVICE, SYSTEM AND METHOD," the full disclosure of which is incorporated herein by reference in its entirety.

### BACKGROUND

The present disclosure relates, in general, to subcutaneous sensor and infusion devices and systems such as, but not limited to infusion sets, injection ports, patch pump devices or other medical devices and systems having at least one subcutaneous sensor interface, and also configured for subcutaneous delivery or communication of infusion media or other fluid. Further examples relate to methods of making and using such devices and systems.

Certain biological conditions may be monitored or sensed, according to modern medical techniques, through one or more analyte sensors inserted subcutaneously from a medical device. For example, blood glucose levels are commonly monitored with subcutaneous sensors, as part of a diabetes treatment. A continuous glucose monitor (CGM) can monitor a patient's blood glucose levels over a period of time. In addition, certain diseases or conditions may be treated by delivering a medication or other substance to the body of a patient, subcutaneously, through an infusion set, injection port or other medical device. For example, diabetes is commonly treated by delivering defined amounts of insulin to the patient at appropriate times. Some patient's with a CGM may require multiple daily injections of insulin.

Subcutaneous sensor devices can include one or more sensor probe, needle or cannula that is configured to be inserted, subcutaneously, through the skin of a patient, to sense or monitor one or more biological conditions.

An infusion set or an injection port device can include one or more cannula configured to be inserted subcutaneously through a patient's skin, to facilitate subcutaneous infusion of a medication or other infusion media. Various examples of infusions sets are described in U.S. Patent Application Publication No. 2018/0318550 (Application No. 15/973,471 and U.S. Patent Application Publication No. 2020/0384187 (Application No. 16/436,486), each of which is incorporated herein by reference, in its entirety.

Some patients employ a sensor device for detecting or monitoring a biological condition or an analyte associated with the condition (such as, but not limited to a blood glucose level) and can also benefit from an infusion set device to deliver infusion media (such as, but not limited to insulin) for treating or responding to a detected or monitored biological condition or analyte. However, it can be inconvenient and uncomfortable to the patient, to install and employ two devices (a sensor device and an infusion set device), each having one or more subcutaneous members.

Accordingly, certain example medical devices as described herein may include one or more sensors and one or more infusion cannula in a single medical device, configured to facilitate sensing or monitor one or more biological conditions or analytes, as well as subcutaneous infusion of a medication or other infusion media. In some examples, each infusion cannula and each sensor is configured for subcutaneous insertion in adjacent, but spaced insertion locations at an insertion site. In other examples, at least one sensor and at least one infusion cannula are configured for combined insertion through a single inserter needle in a single location at the insertion site. By employing one device that includes a sensor for detecting one or more biological conditions or analytes and an infusion cannula for infusing an infusion media in one device as described herein, the overall footprint of the device that the patient is wearing can be reduced, and/or the number of needle insertions can be reduced. Those aspects can yield a reduction in foreign body response and/or can produce few sites with scarring in the hypodermis.

However, for medical devices configured as described herein (to deliver an infusion media at or near the subcutaneous site of the sensor element), it can be beneficial to reduce or minimize interference by the infusion media with the sensor operation. For example, stabilizers in insulin (or in other infusion media) can interfere with the sensor signal. In addition, the volume of insulin (or other infusion media) infused during a bolus may dilute the local tissue glucose (or other parameter), causing sensor signal decay. One or more of those effects are referred to herein as interference effects. Accordingly, certain examples described herein are configured to provide improved separation or isolation of the sensor from the infusion cannula, to reduce or minimize interference effects and provide improved operation, while allowing the device to deliver an infusion media at or near the same insertion location as the sensor. Particular examples described herein provide additional advantages and overcome problems that would otherwise be encountered in arranging a sensor and an infusion cannula in the same device or inserting a sensor element and an infusion cannula in a single inserter needle.

### SUMMARY

A medical device according to certain examples described herein includes a base having a first surface configured to be secured to a patient's skin. A first insertable member is secured to the base and has a length portion extending from the first surface of the base to a distal end of the first insertable member, for insertion through the patient's skin at an insertion site when the first surface of the base is secured to the patient's skin. A second insertable member is configured to be secured to the base and having a length portion extending from the first surface of the base to a distal end of the second insertable member, for insertion through the patient's skin at the insertion site when the first surface of the base is secured to the patient's skin. The first insertable member includes a sensor member for sensing a biological analyte corresponding to a biological condition. The second insertable member includes an infusion cannula for infusing an infusion media. The distal end of the first insertable member and the distal end of the second insertable member are spaced apart by a first distance of at least 5 mm, for reducing interference of the infusion media from the infusion cannula with an operation of the sensor member.

In further examples of the medical device, the first insertable member and the second insertable member are spaced apart from each other by a second distance of at least along a plane of the first surface of the base, for insertion in separate, spaced insertion locations, where the second distance is less than the first distance.

In further examples of the medical device, the first insertable member and the second insertable member are arranged adjacent each other for insertion together in a single insertion location.

In further examples of the medical device, the length portions of the first insertable member and the second insertable members are attached to each other.

In further examples of the medical device, the sensor member has a first length extending from the first surface of the base to the distal end of the sensor member, and the infusion cannula has a second length extending from the first surface of the base to the distal end of the infusion cannula, and wherein the first length is different than the second length.

In further examples of the medical device, the sensor member and the infusion cannula are spaced apart from each other along a plane of the first surface of the base by less than the first distance.

In further examples of the medical device, the first length is greater than the second length, the sensor member has a first surface that faces toward the infusion cannula and that is connected to the infusion cannula, and the sensor member has at least one electrode on the first surface of the sensor member for interfacing with biological fluid or tissue after the sensor member is inserted at the insertion site.

In further examples of the medical device, the sensor member has a first surface that faces toward the infusion cannula and a second surface that faces in an opposite direction as the first surface of the sensor member, and the sensor member has at least one electrode on the second surface of the sensor member for interfacing with biological fluid or tissue after the sensor member is inserted at the insertion site.

In further examples of the medical device, the infusion cannula has fluid flow lumen along an axial length dimension of the infusion cannula, and at least one side wall opening is in fluid flow communication with the lumen for expelling infusion media through a side wall of the infusion cannula. Each side wall opening is provided on a side of the infusion cannula that faces away from the sensor member.

In further examples of the medical device, the length portion of at least one of the first and second insertable members extends from the first surface of the base at an oblique angle relative to the first surface of the base.

In further examples of the medical device, the length portion of each of the first and second insertable members extends from the first surface of the base at an oblique angle relative to the first surface of the base.

Further examples of the medical device include an inserter needle having a hollow channel along a lengthwise axial dimension of the inserter needle, wherein the sensor member and the infusion cannula are arranged adjacent each other in the hollow channel of the inserter needle, for insertion together at a single insertion location.

In further examples of the medical device, the infusion cannula has a first side that has a reduced radius or flat surface facing the sensor member, and the infusion cannula is attached to the sensor member along at least a portion of the first side of the infusion cannula by one or more of an adhesive or heat staking.

In further examples of the medical device, the inserter needle has a slot along its lengthwise axial dimension, and wherein a portion of the infusion cannula extends at least partially into the slot.

In further examples of the medical device, the inserter needle is configured to slide in a direction of its lengthwise axial dimension relative to the sensor member and to the infusion cannula, for selectively withdrawing the inserter needle relative to the sensor member and the infusion cannula, and at least one of the inserter needle or the infusion cannula includes a coating or layer for reducing friction between the inserter needle and one or both of the infusion cannula and the sensor member.

In further examples of the medical device, the base has a channel through which the inserter needle extends for insertion of the sensor member and the infusion cannula.

In further examples of the medical device, the infusion cannula is connected in fluid flow communication with the channel in the base, and the base includes at least one septum located adjacent or within the channel in the base, through which the inserter needle extends for insertion of the sensor member and the infusion cannula.

In further examples of the medical device, the at least one septum provides a port on the base for receiving a needle or rigid cannula of an infusion media source that provides infusion media to the infusion cannula.

Further examples relate to a system including the above medical device, and further including at least one infusion media source including a syringe or other fluid dispenser having a needle through which fluid is dispensed, or a connector hub having a rigid cannula configured to extend through the septum to connect a fluid channel in the connector hub in fluid flow communication with the port on the base, where the fluid channel in the connector hub is connected in fluid flow communication, through a tubing, to a pump and a reservoir of infusion media.

In further examples of the medical device, the biological analyte is at least one of glucose, ketone or lactose.

Further disclosed herein is a medical device which includes a base having a first surface to be secured to a patient's skin. A first insertable member has a length portion extending from the first surface of the base for insertion. A second insertable member has a length portion extending from the first surface of the base for insertion through the patient's skin. The first insertable member includes a sensor member for sensing a biological condition, and the second insertable member includes an infusion cannula for infusing an infusion media. The distal end of the first insertable member and the distal end of the second insertable member are spaced apart by a first distance of at least 5.0 mm, for reducing interference of the infusion media from the infusion cannula with an operation of the sensor member.

### BRIEF DESCRIPTION OF THE DRAWINGS

The above and other aspects and features of the present invention will become more apparent to those skilled in the art from the following detailed description of the example embodiments with reference to the accompanying drawings, in which:
FIG. 1 is a perspective view of an example of a medical device.
FIG. 2 is a cross-section view of an example of the medical device of FIG. 1.
FIG. 3 is a cross-section view of another example of the medical device of FIG. 1.
FIG. 4 is a cross-section view of another example of the medical device of FIG. 1.
FIG. 5 is a cross-section view of another example of a medical device.
FIGS. 6-8 are side views of example configurations of subcutaneous members of the medical device of FIG. 5.
FIG. 9 is a schematic diagram representing cannula lengths and electrode arrangements to explain example configurations of subcutaneous members of the medical device of FIG. 5.
FIGS. 10, 11 and 13-16 are cross-section views of example configurations of subcutaneous members of the medical device of FIG. 5 in hollow insertion needles.
FIG. 12 is a perspective view of a portion of an example configuration of the subcutaneous members that may be used with certain examples of medical devices.
FIG. 17 is a cross-section view of another example configurations of a subcutaneous member of the medical device of FIG. 5 in hollow inserter needles.
FIG. 18 is a schematic diagram representing an orientation of a subcutaneous member, relative to a patient's Langer lines.
FIG. 19 is another schematic diagram representing an orientation of a subcutaneous member, relative to a patient's Langer lines.
FIG. 20 is another schematic diagram representing an orientation of a subcutaneous member, relative to a patient's Langer lines.
FIG. 21 is a perspective view of a portion of the subcutaneous member having a tapered distal end.
FIG. 22 is a perspective view of a portion of the subcutaneous member of FIG. 21 within the inserter needle of FIG. 17.
FIG. 23 is a perspective view of a portion of a further example of a subcutaneous member having an insertion needle in its lumen.
FIG. 24 is a perspective view of a portion of the subcutaneous member of FIG. 23, without the insertion needle.
FIG. 25 is a side, cross-section view of a medical device having a subcutaneous member and an insertion needle.
FIG. 26 is a side, perspective view of the medical device of FIG. 25, with the needle hub separated from the base.

### DETAILED DESCRIPTION

Hereinafter, example embodiments will be described in more detail with reference to the accompanying drawings. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art. Accordingly, processes, elements, and techniques that are not necessary to those having ordinary skill in the art for a complete understanding of the aspects and features of the present invention may not be described. Unless otherwise noted, like reference numerals denote like elements throughout the attached drawings and the written description, and thus, descriptions thereof may not be repeated. Further, features or aspects within each example embodiment should typically be considered as available for other similar features or aspects in other example embodiments.

Certain terminology may be used in the following description for the purpose of reference only, and thus are not intended to be limiting. For example, terms such as "top", "bottom", "upper", "lower", "above", and "below" could be used to refer to directions in the drawings to which reference is made. Terms such as "front", "back", "rear", "side", "outboard", and "inboard" could be used to describe the orientation and/or location of portions of the component within a consistent but arbitrary frame of reference which is made clear by reference to the text and the associated drawings describing the component under discussion. Such terminology may include the words specifically mentioned above, derivatives thereof, and words of similar import. Similarly, the terms "first", "second", and other such numerical terms referring to structures do not imply a sequence or order unless clearly indicated by the context.

It will be understood that when an element or feature is referred to as being "on," "secured to," "connected to," or "coupled to" another element or layer, it can be directly on, connected to, or coupled to the other element or feature, or one or more intervening elements or features may be present. In addition, it will also be understood that when an element or features is referred to as being "between" two elements or features, it can be the only element or feature between the two elements or features, or one or more intervening elements or features may also be present.

The terminology used herein is for the purpose of describing particular embodiments and is not intended to be limiting of the present invention. As used herein, the singular forms "a" and "an" are intended to include the plural forms as well, unless the context clearly indicates otherwise. It will be further understood that the terms "comprises," "comprising," "includes," and "including," "has, " "have, " and "having," when used in this specification, specify the presence of the stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent variations in measured or calculated values that would be recognized by those of ordinary skill in the art. Further, the use of "may" when describing embodiments of the present invention refers to "one or more embodiments of the present invention." As used herein, the terms "use," "using," and "used" may be considered synonymous with the terms "utilize," "utilizing," and "utilized," respectively.

Unless otherwise defined, all terms (including technical and scientific terms) used herein have the same meaning as commonly understood by one of ordinary skill in the art to which the present invention belongs. It will be further understood that terms, such as those defined in commonly used dictionaries, should be interpreted as having a meaning that is consistent with their meaning in the context of the relevant art and/or the present specification, and should not be interpreted in an idealized or overly formal sense, unless expressly so defined herein.

Example embodiments relate to medical devices having one or more subcutaneous sensor probe, cannula or needle connected to sensor electronics, and one or more infusion cannula for delivery or communication of an infusion media or other fluid. In certain examples, the infusion media is an insulin or insulin formulation. In other examples, the infusion media is another drug, medication, or other fluidic media. In certain examples, the sensor probe, cannula or needle and the sensor electronics are configured to sense or monitor blood glucose. In other examples, the sensor probe, cannula or needle and the sensor electronics are configured to sense or monitor one or more other analytes (including but not limited to ketone or lactose) or biological conditions. In certain examples, the sensor probe, cannula or needle (each referred to herein, as the sensor element) and the infusion cannula are arranged to extend from a base of the medical device, and may be located adjacent, but spaced from each other, to be inserted in separate, spaced insertion locations at an insertion site under the base. In other examples, the sensor element and the infusion cannula are arranged on the base of the medical device, to be inserted, together, in a single insertion location.

In particular examples, the sensor element, or the infusion cannula (or both) may be configured to be relatively flexible, for example, to flex with movement of adjacent tissue when in an inserted state. In those examples, a separate, rigid inserter needle may be used to facilitate inserting the flexible sensor element, or the flexible infusion cannula (or both) through the patient's skin, to a subcutaneous operating position. In examples in which the sensor element and the infusion cannula are arranged adjacent, but spaced from each other, the sensor element and the infusion cannula may employ separate inserter needles.

In examples in which a flexible sensor element and a flexible infusion cannula are arranged to be inserted in a single insertion location, the sensor element and the infusion cannula may be inserted simultaneously, with a single inserter needle. Examples employing a single inserter needle for simultaneous insertion of the sensor element and an infusion cannula can provide advantages, including improved patient comfort and simplification of insertion procedures (by reducing the number of insertions needed to place the sensor element and the infusion cannula to a single insertion).

In certain examples, the medical device is at least one of an infusion set, an injection port, a patch pump device or other medical device for delivery or communication of an infusion media or other fluid to (or from) the patient, and also has at least one subcutaneous sensor for detection or monitoring of an analyte or biological condition of the patient. Examples in which the medical device comprises an infusion set may be configured to connect (or are connected) in a system, through one or more fluid flow tubing to an infusion media source (such as, but not limited to a reservoir containing an infusion media, a controlled infusion media pump, or the like), to provide individual, intermittent or continuous delivery of infusion media to the patient, through the infusion cannula. Examples that comprise a patch pump may include a pump device and a reservoir supported on the same base from which the sensor element and the infusion cannula extend.

Examples that comprise an injection port may include a port having a septum configured to be pierced by a needle of a syringe, smart pen or other external fluid injector, and to receive fluid injected from the syringe, smart pen or other injector, for delivery to the patient through the infusion cannula. In some examples, the medical device may include a combination of an infusion set and an injection port, or a combination of a patch pump and an injection port. In particular examples, the medical device is configured to permit multiple sensor detections or continuous monitoring, as well as one or more (or multiple) injections of medication into a patient without the need to re-puncture the patient's skin. Other examples relate to methods of making and using such medical devices and medical systems.

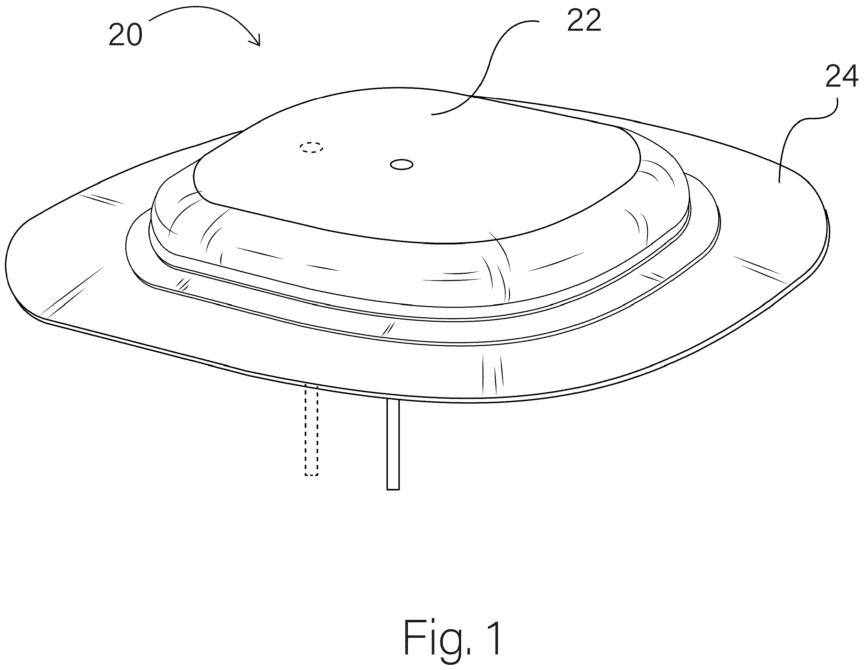

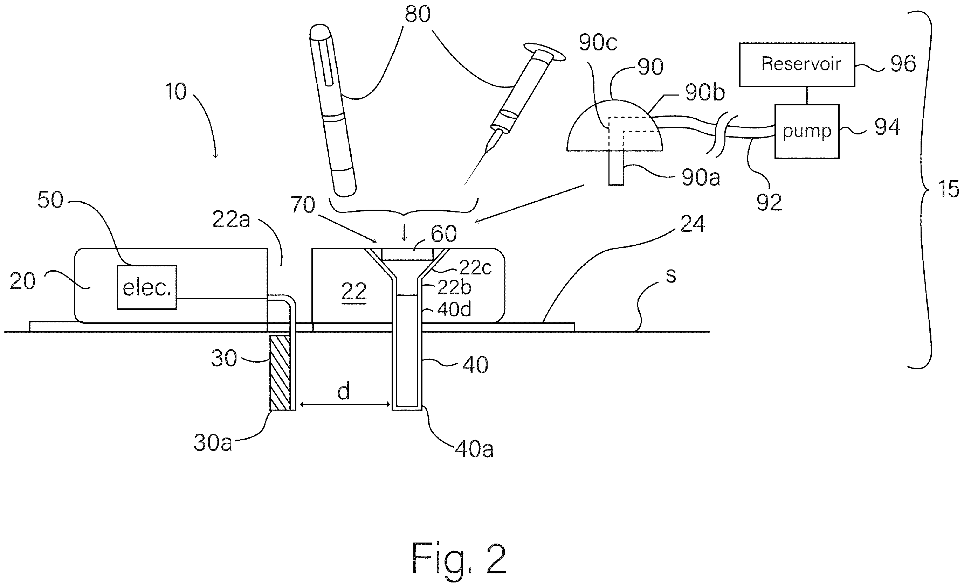

An example of a medical device 10 is shown in a top, perspective view in FIG. 1. In certain examples, the medical device 10 includes first and second insertable members 30 and 40 that are spaced apart from each other (as shown with the broken line representation of a second insertable member in FIG. 1). In other examples, the medical device includes first and second insertable members that are arranged together (not spaced apart) to be inserted in a single insertion location (where the broken line representation of the spaced-apart insertable member in FIG. 1 would be omitted). One example configuration of the medical device 10 (and a system 15 that includes the medical device 10) is shown in a cross-section, schematic view in FIG. 2, attached to an epidermal surface S of a patient. The medical device 10 includes a base 20 that has a bottom surface (the downward-facing or skin-facing surface in FIG. 2). The base 20 may include a disc-shaped body 22 (or a body of any other suitable shape) and a patch or flange 24 on the bottom surface of the body 22 for contacting and adhering to the epidermal surface S of a patient, during use. The flange 24 may be fixed to the body 22 of the base by any suitable connection mechanism, or may be formed integral with the body 22 of the base. The body 22 of the base 20 may be made of any generally rigid material such as, but not limited to plastic, metal, ceramic, composite material, or any combinations thereof. The flange 24 may be made of one or more of such generally rigid materials, or may be made of a more flexible material such as, but not limited to a silicon rubber or other elastic polymer, a natural or synthetic fabric, or the like.

In some examples, the flange 24 includes an adhesive material layer on its skin-facing side (the downward-facing side in FIGS. 1 and 2) that may be selectively exposed to adhere the base 20 to the epidermal surface S, at a desired insertion site. In certain examples, a backing or release material layer (not shown) is adhered to the adhesive material layer to cover and protect the adhesive material layer from exposure to dirt or other environmental contaminants before adhering the base 20 to a patient's skin. In those examples, the release material layer is selectively removable from the adhesive material layer to expose the adhesive material for adhering the base 20 to the epidermal surface S. In other examples, the adhesive material layer and the release material layer may be omitted. Alternatively or in addition, in further examples, the base 20 may include other suitable mechanisms for securing the base 20 to a patient's skin including, but not limited to bands, straps, sutures, suture eyelets for receiving sutures, or combinations thereof.

The medical device 10 further includes a first subcutaneously insertable member 30 and a second subcutaneously insertable member 40 secured to and supported by the base 20. The first insertable member 30 may include a sensor element (i.e., a probe, a needle, a cannula, or the like, of a sensor). The first insertable member 30 has a distal end 30a that is configured to be inserted through the epidermal surface S, to a subcutaneous depth location. The proximal end of the sensor element 30 is operably connected to sensor electronics 50 located in the base 20. The sensor electronics may be any suitable electronics that is configured to detect or monitor electrical parameters or electrical conditions of one or more electrodes located on the sensor probe, needle or cannula (where the electrical parameters or conditions are associated with one or more biological conditions or analytes being detected or monitored). In other examples, the sensor electronics may be configured to obtain one or more samples of a biological fluid from the sensor needle or cannula and detect or monitor one or more biological conditions or analytes.

The second insertable member 40 includes an infusion cannula composed of a tubing having a fluid flow channel along its length. In some examples, the infusion cannula 40 is open on its distal end 40a (the end facing downward in FIG. 2), to allow fluid flowing through the fluid flow channel to exit the infusion cannula 40 at its distal end. In other examples, as described below, the side wall of the infusion cannula 40 may have one or more openings to the fluid flow channel, to allow fluid flowing through the fluid flow channel to exit the infusion cannula 40, laterally through the side wall opening(s). In certain examples in which the cannula side wall has one or more openings, the infusion cannula 40 may be sealed at its distal end to allow fluid flowing through the fluid flow channel to exit only through the side wall openings. In other examples, the infusion cannula 40 may be open at its distal end and have one or more side wall openings, to allow fluid flowing through the fluid flow channel to exit both the distal end and the side wall opening(s) of the infusion cannula 40.

The body 22 of the base 20 has a first channel 22a from which the first insertable member 30 (e.g., the sensor element) extends. In particular examples, the first channel 22a provides a passage through the base 20, through which an inserter needle may be passed to facilitate subcutaneous insertion of the first insertable member 30, and to withdraw or remove the inserter needle after insertion of the first insertable member 30. In some examples, a hollow inserter needle may be employed, and the first insertable member 30 is located within the hollow needle before and during insertion. Once inserted, the inserter needle may be withdrawn from the first insertable member 30, through the first channel 22a, leaving the first insertable member 30 in an inserted state.

A second channel 22b extends through the body 22. The infusion cannula 40 is secured to the second channel 22b by any suitable mechanism such as, but not limited to adhesives, heat staking, or other mechanical connection. In the example in FIG. 2, a portion 40b of the cannula 40 extends into the second channel 22b and partially through the body 22 of the base 20. In some examples, a proximal (or upper) portion of the second channel 22b is flared outward to form a cone-shaped recess 22c at the proximal opening of the channel 22b. In some examples, the proximal end of the cannula 40 be flared outward as well. The cone-shaped recess 22c may be formed integral with the body 22 of the base 20. In other examples, the cone-shaped recess 22c is formed by a cone-shaped guard member made of metal or other rigid material, attached to the body 22 of the base 20 at the proximal end of the channel 22b.

In particular examples, the second channel 22b provides a passage through the base 20, through which an inserter needle may be passed to facilitate subcutaneous insertion of the cannula 40, and to remove the inserter needle after insertion of the cannula 40. The cone-shaped recess 22b can facilitate guiding of a needle toward the cannula 40. In some examples, the inserter needle may extend through the cannula 40 and has a sharp tip extended out of the distal end of the cannula 40, before and during insertion of the cannula. In other examples, a hollow inserter needle may be employed, and the cannula 40 is located within the hollow needle before and during insertion of the cannula. Once inserted, the inserter needle may be withdrawn from the cannula 40, through the second channel 22b, leaving the cannula 40 in an inserted state.

A septum 60 is located within the cone-shaped recess 22c, to provide a fluid seal at the proximal end of the channel 22b. A similar septum (not shown) may be locate in or adjacent the channel 22a in the base 20. The septum 60 may be a pierceable member, made of a material that is configured to provide a fluid seal on or in the channel 22b, and to be selectively pierced by the inserter needle, a needle of an external infusion media source, or by a rigid cannula of a connector hub (or any combination thereof). The septum 60 is configured to provide a fluid seal around the needle or rigid cannula, and to reseal after removal of the needle or rigid cannula. The septum 60 may be made of any suitable material for such purposes, such as, but not limited to silicon rubber, polyurethane, or other elastic polymer, or the like. In some examples, the septum 60 may be pre-pierced or formed with a slot or cut that is configured to self-seal around a needle or rigid cannula, and to self-seal after removal of the inserter needle or rigid cannula.

The septum 60 and the proximal end of the channel 22b of the base 20 form a port 70, into which a needle or a rigid cannula may be selectively inserted as described below. The port 70 also provides a passage through which an inserter needle may be selectively inserted and withdrawn to facilitate insertion of the infusion cannula 40. In examples in which a similar septum is located in or adjacent the channel 22a of the base 20, that septum and the channel 22a form a further port for a further inserter needle to facilitate insertion of the sensor member 30.

In certain examples, the port 70 is an infusion port, configured to selectively receive a needle of a syringe, a needle of a smart pen injector, a needle of an IV bag delivery device, or a needle of another infusion media source (referred to as an external source of infusion media 80 in FIG. 2). When the needle of the external source of infusion media is received in the port 70 (by piercing the needle through the septum 60), infusion media from the external source may be received in the proximal end of the infusion cannula 40, and may flow through the channel of the cannula 40 and be expelled into the patient, through one or more openings at the distal end 40a or in the side wall of the cannula 40. In that regard, the port 70 may be configured as an infusion port, for receiving a needle of an external source of an infusion media, and administering a defined or desired volume of infusion media to the patient from the external source.

In another example, the port 70 may be configured to receive a rigid cannula of a connector hub 90. More specifically, the connector hub 90 may include a body made of a generally rigid material, such as, but not limited to a plastic, metal, ceramic, composite or combination thereof, and may have a generally rigid cannula or needle 90a extending from a distal side of the body of the hub 90 (the downward side in FIG. 2). The rigid cannula 90a is connected in fluid flow communication with a tubing port 90b, through a fluid flow channel 90c in the hub body 90.

The tubing port 90b is selectively connectable in fluid flow communication, through a flexible tubing 92, to a pump 94 and a reservoir 96 of infusion media. The connector hub 90 is configured to connect to the port 70 on the base 20, by inserting the rigid cannula or needle 90a through the septum 60 in the port 70. In certain examples, a further connection mechanism may be provided to selectively couple the hub body 90 to the body 22 of the base 20 in one or more fixed positions, or in a rotatable position (rotatable about the longitudinal axis of the rigid cannula or needle 90a) relative to the base 20. When the connector hub 90 is connected to the port 70, the infusion cannula 40 is connected in fluid flow communication with the pump 94 and the reservoir 96, through the flexible tubing 92, to receive infusion media from the reservoir 96 at a rate controlled by the pump 94.

In yet other examples, the body 22 of the base 20 may contain a pump 94 and a reservoir 96 that connect in fluid flow communication to the channel 22b, to supply infusion media from the reservoir 96 to the proximal end of the cannula 40. In that configuration, the pump 94 and the reservoir 96 may be included within the base 20 or directly attached to and supported by the base 20, as a patch-pump device. In some examples, the patch-pump device may also include the port 70 for selectively receiving a needle of an external infusion media source, as described above.

In the example in FIG. 2, the first and second insertable members 30 and 40 (corresponding to the sensor element and the infusion cannula, respectively) extend from the base 20 at approximately equal lengths from the base 20. Accordingly, when subcutaneously inserted in a patient, the first and second insertable members 30 and 40 will extend to approximately equal subcutaneous depths, as shown in FIG. 2. In other examples, the first insertable member 30 may have a longer length than the second insertable member 40, to extend to a deeper subcutaneous depth than the second insertable member 40, when inserted in the patient. In yet other examples, the second insertable member 40 may have a longer length than the first insertable member 30, to extend to a deeper subcutaneous depth than the first insertable member 30, when inserted in the patient. In examples described herein, the lengths of the first and the second insertable members 30 and 40 is measured from the bottom surface (skin-facing surface) of the base 20 to the distal ends 30a and 40a of the members 30 and 40 (i.e., the length extending from the base 20).

As described herein, the infusion cannula (or second insertable member) 40 is configured to receive an infusion media from an infusion media source and expel the infusion media from an open distal end or from one or more side wall openings (or both) of the infusion cannula, when the infusion cannula is in an inserted state as shown in FIG. 2. The infusion media expelled from the infusion cannula can tend to form (at least temporarily) a depot or volume of high concentration of infusion media in the tissue at and around the subcutaneous opening(s) of the infusion cannula. However, in particular examples, a distance d between the first and second insertable members 30 and 40 may be selected to avoid or minimize interference (from infusion media expelled from the infusion cannula 40) with the accuracy or operation of the first insertable member 30 (e.g., the sensor element) or the accuracy of the sensor electronics to which the sensor element connects. In the example in FIG. 2, the distance d is measured along the plane of the bottom surface of the base 20, or of the epidermal surface S.

According to certain examples described herein, the distance d is selected, based, at least partially, on one or more of the following factors: the length (or subcutaneous depth) of the first insertable member 30, the length (or subcutaneous depth) of the second insertable member 40, the difference in lengths (or difference in subcutaneous depths) of the first and the second insertable members 30 and 40, the type and configuration of the sensor, the configuration of the infusion cannula, the type of infusion media to be expelled, the rate of flow of infusion media from the second insertable member 40, and the volume of infusion media expected to be expelled.

For example, in a medical device configured for insulin infusion and blood glucose detection or monitoring, and having a first insertable member 30 (the sensor element) and a second insertable member 40 (the infusion cannula) that are each about the same length (such as, but not limited to 10 mm long), the distance d between the first and second insertable members may be configured to be about 11 mm. In other examples, that distance d may be configured to be greater than 11 mm. In yet other examples, that distance d may be less than 11 mm, such as in contexts in which the interference of the infusion media with the sensor element is mitigated in other ways or is not significant.

In yet other examples including, but not limited to insulin delivery and blood glucose sensing, the length of the first insertable member 30 (the sensor element) may be greater than 10 mm or less than 10 mm, or the length of the second insertable member 40 (the infusion cannula) may be greater than 10 mm or less than 10 mm.

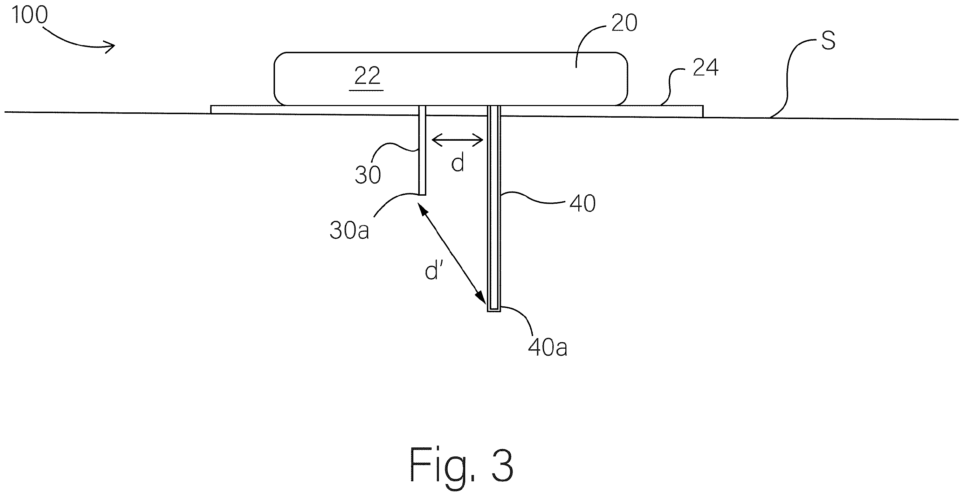

In further examples, as shown in FIG. 3, a medical device 100 may include a base 20 configured according to the examples described above with regard to the base 20 in FIG. 2. The medical device 100 also includes first and second subcutaneous members 30 and 40 corresponding to those discussed above with regard to FIG. 2. However, in the example in FIG. 3, the length of the infusion cannula 40 is greater than the length of the insertable member of the sensor 30, such that a distance d' between the insertable member of the sensor 30 and the open distal end of the infusion cannula 40 is greater than the distance d.

Thus, with regard to the example described above with regard to FIG. 2, where the distance d was selected to be about 11 mm, that same distance of about 11 mm can be achieved for d' in FIG. 3, while allowing the spacing d between the first and second subcutaneous members 30 and 40 to be much smaller than that of FIG. 2. For example, to achieve a distance d' of 11 mm, it may be possible to configure the base 20 in FIG. 3 to have a distance d of as small as about 5 mm (or larger or smaller than 5 mm, depending upon the distance d'). Accordingly, by increasing the difference in lengths of the first and second subcutaneous members 30 and 40 (to increase the distance d'), the distance d between the first and second subcutaneous members 30 and 40 can be reduced. In that regard, the size or foot print of the base 20 may be reduced, as the distance d is reduced. While a distance d' of 11 mm is used in the above examples, other examples may provide a distance d' of other suitable lengths, including, but not limited to a distance d' of at least 5.0 mm, or a distance d' in the range of about 2 mm to about 7 mm.

In the examples shown in FIGS. 2 and 3, the first and second subcutaneous members 30 and 40 are shown as extending generally perpendicularly, relative to the plane of the distal (bottom) surface of the base 20, or the plane of the subcutaneous surface S. However, in other examples, one or both of the first and second subcutaneous members 30 and 40 may extend at an oblique angle (non-parallel, non-perpendicular angle) relative to the plane of the bottom surface of the base 20, or the plane of the subcutaneous surface S, for angled insertion into the epidermal. As compared to the example in FIG. 2, an angled insertion direction may allow the second subcutaneous member (i.e., the infusion cannula) 40 to have a longer length, or a reduced insertion depth below the surface S (or both) and, thus, can increase the distance d' or can reduce discomfort to the patient (or both).

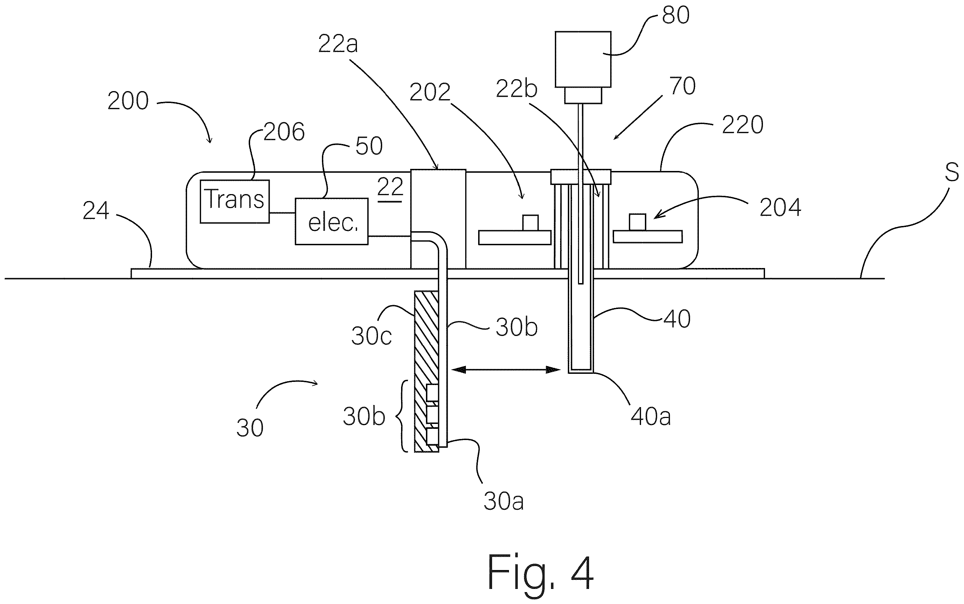

In further examples, as shown in FIG. 4, a medical device 200 may include a base 20 configured according to any of the examples described above with regard to the base 20 in FIG. 2 or of FIG. 3. The medical device 200 also includes first and second subcutaneous members 30 and 40 corresponding to those discussed above with regard to FIG. 2. However, in the example in FIG. 4, the first subcutaneous member 30 is longer than the second subcutaneous member 40, and further details of a sensor member corresponding to the first subcutaneous member 30 are shown. In particular, the sensor member 30 has an electrode arrangement 30b on a substrate 30c. The sensor substrate 30c may be a flexible strip of supporting material, and has one or more electrical conductors along a portion of its length extending to the electrode arrangement 30b. The sensor substrate 30c may be made to have a relatively thin dimension, to minimize discomfort to the patient during or after insertion. In some examples, the electrodes or a portion of the sensor substrate (or both) have a layer 30d of glucose oxidase, or other suitable material (chemistry layer).

The electrode arrangement 30b may comprise one or more (or a plurality of) electrodes, such as, but not limited to the examples described below. The one or more electrodes are electrically coupled to the electronics 50 in the base 20. In particular examples, the electrode arrangement 30b includes at least one working electrode (WE), at least one reference electrode (RE) and at least one control electrode (CE). For example, the WE may be configured to provide a positive current response, when glycerin reaches that electrode. In other examples, the electrode arrangement 30b may have other configurations of one or more electrodes. The one or more electrodes of the electrode arrangement 30b may be configured to be in electrical contact with biological fluid or tissue, when inserted in the patient, for detection or monitoring of a biological condition or analyte. In particular examples, the electrode arrangement is configured for detection or monitoring of the patient's blood glucose level. In other examples, the electrode arrangement is configured for detection of one or more other biological conditions or analytes.

In the example in FIG. 4, the electrodes of the electrode arrangement 30b are provided on one side of the sensor substrate 30c. In that example, the electrodes are on the side of the sensor substrate 30c that faces away from (in the opposite direction of) the infusion cannula 40. By arranging the electrodes of the electrode arrangement 30b on that side of the sensor substrate 30c, the electrodes may be directed to face away from the infusion cannula 40, which can help increase the separation or isolation of the sensor electrode arrangement 30b from the infusion media outlet of the infusion cannula 40.

In the example shown in FIG. 4, the infusion cannula 40 has an axial length that is smaller than the axial length of the first subcutaneous member 30. In other examples, the infusion cannula 40 in FIG. 4 may have an axial length equal to that of the first subcutaneous member 30 as described with regard to FIG. 2, or may be provided with a longer axial length than the axial length of the first subcutaneous member 30 to increase the distance d' or to decrease the distance d as described above with regard to FIG. 3. Alternatively or in addition, the example in FIG. 4 may be implemented with an angled insertion direction, for example, to allow for a greater separation or isolation distance or improved patient comfort (or both), as described above with regard to further embodiments of the example in FIG. 3.

In any of the examples described herein, the infusion cannula 40 may be configured with one or more openings through the side wall of the cannula, for expelling infusion media, as shown in FIG. 45. In particular examples, the one or more openings 40c (one shown in FIG. 54) are on the side of the infusion cannula 40 that faces away from (in the opposite direction of) the first subcutaneous member 30. In some examples, the distal end of the infusion cannula 40 may be closed to allow the infusion media to be expelled only from the one or more side wall openings 40c, and not from the distal end of the cannula 40. In other examples, the distal end 40a of the infusion cannula 40 may also have an opening, in addition to the one or more side wall openings 40c, to allow some, but not all of the infusion media to be delivered from the side wall openings and some, but not all of the infusion media to be delivered from the distal end. In some examples, the distal end 40a may be tapered to a relatively small opening, to reduce the percentage of infusion media that flows out of the distal end 40a relative to the one or more side wall openings 40c. By arranging the opening(s) 40c on that one side of the infusion cannula 40, the opening(s) 40c may be directed to face away from the first subcutaneous member 30, such that infusion media is expelled entirely (or partially) in the direction away from the first subcutaneous member 30, to help increase the separation or isolation of the first subcutaneous member 30 from the infusion media outlet of the infusion cannula 40.

In any of the examples described herein, it may be desirable to detect whether or not a needle from an external source or the rigid cannula of a connector hub is fully or properly inserted into the port 70. Accordingly, any of examples described herein may include one or more detection mechanisms in or associated with the base 20. An example detection mechanism is shown in FIG. 4 as including an infra-red (IR) or other optical emitter 202 and a corresponding IR or other optical detector 204 arranged on opposite sides of the channel 22b, to detect the presence of a needle in the channel 22b (between the optical emitter and the optical detector). In those examples, the base 20 may include a transparent or partially transparent wall or windows around the channel 22b, through which an optical beam from the optical emitter 202 can align with and be received by the optical detector 204. In those examples, the base 20 may include other electronics coupled with the detection mechanism, such as, but not limited to a battery or other power source (not shown) for providing electrical power to the electronics, and a transmitter device (206) for transmitting a signal corresponding to the detection of a needle or rigid cannula in the channel 22b to an external processor system. The external processor system may be configured to confirm, track or verify full or proper needle insertions (or improper insertions), for monitoring or managing a treatment program. While the example in FIG. 4 shows a detection mechanism having an IR (or other optical) emitter and detector, other examples may include other detection mechanisms including, but not limited to magnetic detection, inductive detection, mechanical detection or other detection mechanisms.

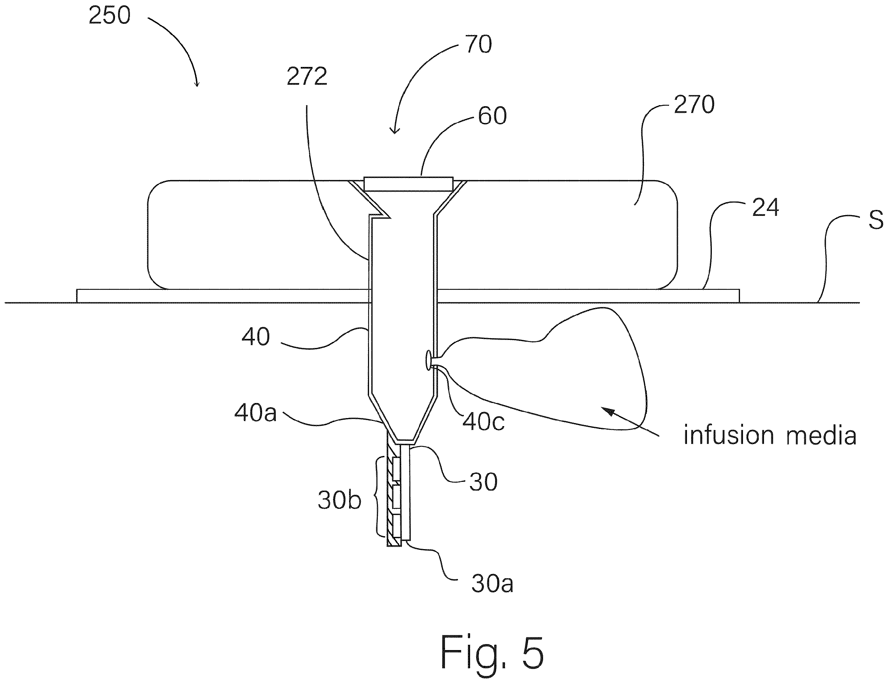

In the examples of FIGS. 2-4, the medical device includes a base from which two subcutaneous members 30 and 40 extend adjacent, but spaced from each other, for insertion in two insertion locations at an insertion site under the base. In further examples, as described with regard to FIGS. 5-14, two subcutaneous members (e.g., the sensor member 30 and the infusion cannula 40) may be arranged together, to be inserted simultaneously in one insertion location at the insertion site. A single insertion of the subcutaneous members 30 and 40, together, can be beneficial by reducing the number of insertions required, and by allowing the size or footprint of the medical device to be smaller, as compared to examples (as in FIGS. 2-4) in which the subcutaneous members 30 and 40 are arranged adjacent, but spaced from each other. A separation distance d' between the distal ends of the subcutaneous members 30 and 40 (or between the sensor electrode arrangement on the sensor member 30 and the infusion media outlet of the infusion cannula 40) can be accomplished in various manners as described herein.

More specifically, with reference to the further example shown in FIG. 5, a medical device 250 may include a base 270 configured according to any of the examples of the base 20 described above in FIGS. 2, 3 or 4, but has one channel 272 for accommodating a single inserter needle (not shown in FIG. 5). The medical device 250 also includes first and second subcutaneous members 30 and 40 corresponding to those discussed above with regard to FIG. 2. However, in the example in FIG. 5, the subcutaneous members 30 and 40 are arranged for insertion, together, with the single inserter needle. The single inserter needle may extend through the channel 272 before and during insertion of the first and second subcutaneous members 30 and 40 and, then, may be axially withdrawn from the first and second subcutaneous members 30 and 40, through the channel 272, leaving the first and second subcutaneous members 30 and 40 in an inserted state.

In the example in FIG. 5, the first and second subcutaneous members 30 and 40 may be secured to the base 270 in the same manner as described above with regard to securing the first and second subcutaneous members 30 and 40 to the base 20. However, in the example in FIG. 5, the first and second subcutaneous members 30 and 40 extend from the same channel 272, such that both the first and second subcutaneous members 30 and 40 may be inserted, together, with a single insertion needle. Accordingly, the first and second subcutaneous members 30 and 40 are in contact (or close proximity) and are not spaced apart by the distance d, discussed above. Therefore, in particular examples, the first and second subcutaneous members 30 and 40 include one or more configurations as described herein, to reduce or minimize interference effects discussed above.

In the example in FIG. 5, the second subcutaneous member 40 includes an infusion cannula that has a side wall opening 40c, as described above with regard to FIG. 4. In addition, the distal end 40a of the infusion cannula 40 is tapered or reduced in diameter, to reduce the outflow rate of infusion media from the opening at the distal end 40a, and increase the outflow rate of infusion media from the side wall opening 40c. In other examples, the distal end 40a of the infusion cannula 40 in FIG. 5 may be open and not tapered or reduced in diameter relative to other portions of the infusion cannula 40. In yet other examples, the side wall opening 40a in FIG. 5 may be omitted. In yet other examples, the distal end 40a of the infusion cannula 40 in FIG. 5 may be closed, such that all of the infusion media is expelled through the side wall opening 40c.

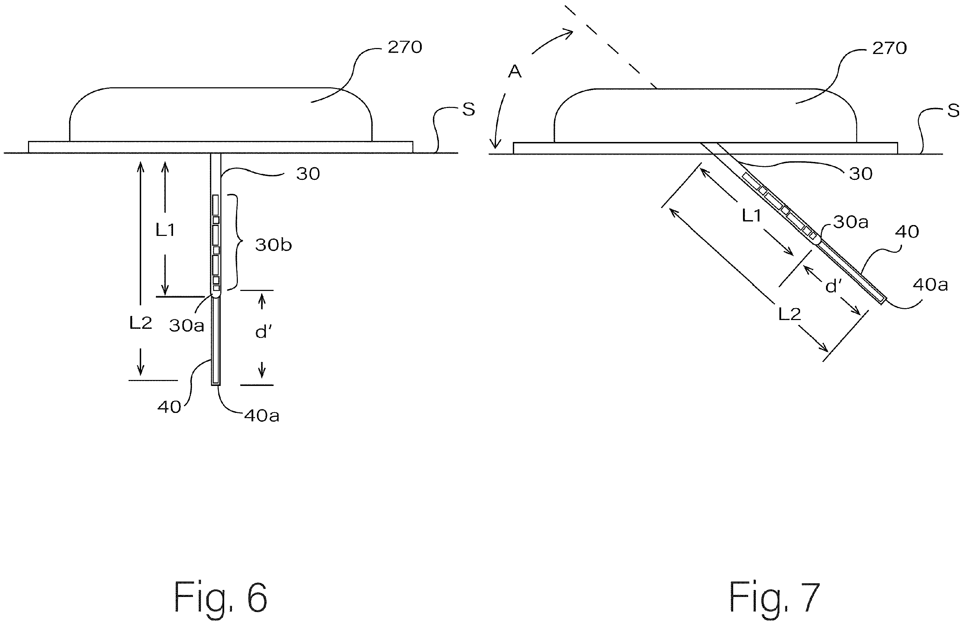

As discussed above, one manner of reducing or minimizing such interference effects includes configuring or selecting the length at which each the first and second subcutaneous members 30 and 40 extends from the base 20 or 270 to increase or maximize a separation distance d'. The drawing of Figs. 6-8 show certain further example configurations of subcutaneous members extending from the base 270 (a portion of which is shown in those drawings) of a medical device corresponding to the medical device 250 of FIG. 5, and are further configured or selected to reduce or minimize interference effects discussed above.

In the example in Fig. 6, the first subcutaneous member 30 includes a sensor member having sensor electrodes 30b for contacting biological fluid or tissue, when the sensor member is in an inserted state. The sensor member 30 has an axial length L₁ from the base 270 to the distal end 30a of the sensor member. In some examples, that axial length L₁ of the sensor member 30 is about 8.5 mm. In other examples, the axial length of the sensor member may be less than 8.5 mm, or may be greater than 8.5 mm.

In the example in Fig. 6, the second subcutaneous member 40 includes an infusion cannula having an axial length L₂ that is longer than the axial length L₁ of the sensor member. The infusion cannula 40 is arranged directly adjacent to (behind, in the view of Fig. 6) the sensor member 30. In particular examples, the infusion cannula 40 and the sensor member 30 in FIG. 6 are in contact with each other, or are attached to each other. Accordingly, in examples in which the electrodes 30b are arranged at or near the distal end 30a of the sensor member 30, and the infusion outlet is located at the distal end 40a of the infusion cannula 40, the difference in length dimensions L₂-L₁ defines a separation distance between the sensor electrodes and the infusion outlet. In particular examples, the length of the infusion cannula 40 or the length of the sensor member 30 (or both) are configured or selected to provide a desired separation distance between the electrodes on the sensor member 30 and the infusion media outlet of the infusion cannula 40, to reduce or avoid interference effects described above.

The difference in length dimensions L₂-L₁ beneficial to provide a desired separation distance d' may depend on (and be determined based on) one or more of the following factors: a desired length (or subcutaneous depth) of the first insertable member 30, a desired length (or subcutaneous depth) of the second insertable member 40, the type and configuration of the sensor, the configuration of the infusion cannula, the type of infusion media to be expelled, the rate of flow of infusion media from the second insertable member 40, and the volume of infusion media expected to be expelled.

Thus, in the above example in which the axial length L₁ of the sensor member 30 is about 8.5 mm, the axial length of the infusion cannula 40 may be selected to be about 14 mm, to provide a desired separation distance d' between the distal ends 30a and 40a of about 5.5 mm. In particular examples, a separation of about 5.5 mm can provide a suitable separation distance to reduce or avoid interference effects described above. In other examples, the axial length of the infusion cannula 40 may be less than 14 mm, or may be greater than 14 mm, and the separation distance d' between distal ends 30a and 40a may be less than 5.5 mm or may be greater than 5.5 mm.

In the example in FIG. 6, the sensor electrodes 30b are arranged on the side of the substrate of the sensor member 30 that faces away from (opposite to) the infusion cannula 40. The cannula 40 is shown in the view of FIG. 6 as being behind the sensor member 30. The electrodes 30b are on the side of the sensor member 30 that faces outward from the sheet in FIG. 6.

The example in FIG. 7 is similar to the example in FIG. 6, except that the sensor member 30 and the infusion cannula 40 are configured to be inserted at an oblique angle relative to the plane of the bottom surface of the base 270, or the plane of the subcutaneous surface S, for angled insertion into the epidermal. An angled insertion direction may allow the infusion cannula to have a relatively long length (as in the example of FIG. 6), but can reduce the depth of insertion below the surface S and, thus, can reduce discomfort to the patient. In particular examples, an angle A of insertion may be about 30 degrees (or 150 degrees) relative to the plane of the epidermal-facing surface of the base 270 (the bottom surface in FIG. 7) or to the plane of the epidermal surface S. In other examples, the angle A of insertion may be any suitable oblique angle.

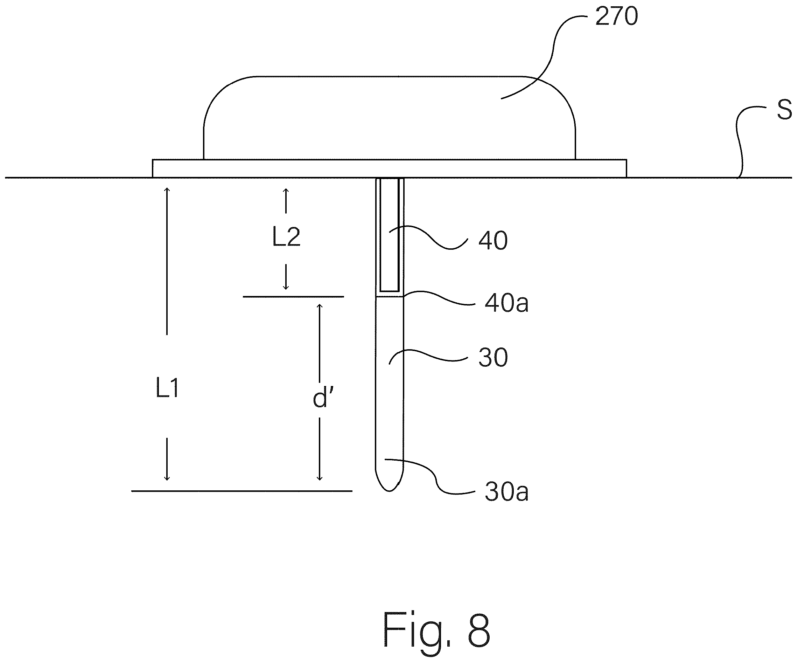

The example in FIG. 8, is similar to the example in FIG. 6, except that the infusion cannula 40 has an axial length L₂ that is shorter than the axial length L₁ of the sensor member 30. In the view of FIG. 8, the sensor member 30 is located behind the infusion cannula 40. The electrodes 30b (not in view in FIG. 8) are on the side of the sensor member 30 that faces into the sheet in FIG. 8 and, thus face away from (opposite to) the infusion cannula 40. In the example in FIG. 8, the infusion cannula 40 may be significantly shorter than the example of the infusion cannula 40 in FIG. 6.

In other embodiments of the example in FIG. 8, the sensor member 30 may be arranged such that the electrodes 30b are on the side of the sensor member 30 that faces toward the direction of the cannula 40 (i.e., faces out of the sheet in FIG. 8). In those examples, the infusion cannula 40 may be configured to be sufficiently shorter than the sensor member 30, to space the infusion media outlet of the infusion cannula 40 from the electrodes on the sensor member 30 a sufficient distance to reduce or minimize interference effects described above.

In one example of the configuration in FIG. 8, the axial length L₁ of the sensor member 30 may be about 8.5 mm, and the axial length of the infusion cannula 40 may be selected to be about 2.5 mm, to provide a desired separation distance d' between 30a and 40a of about 6 mm. In particular examples, a separation of about 6 mm can provide a suitable separation distance to reduce or avoid interference effects described above. In other examples, the axial length of the infusion cannula 40 may be less than 2.5 mm, or may be greater than 2.5 mm, and the separation distance d' between distal ends 30a and 40a may be less than 6 mm or may be greater than 6 mm. As described above, particular examples include other separation distances suitable for reducing or avoiding interference effects. While the drawing in FIG. 8 shows an example having a perpendicular angle of insertion, other examples of the configuration in FIG. 8 may be configured to be inserted at an oblique angle relative to the plane of the bottom surface of the base 270, or the plane of the subcutaneous surface S, for angled insertion into the epidermal.

Accordingly, as shown in FIGS. 6-8, certain examples described herein include an infusion cannula 40 that is longer than the sensor member 30, while other examples described herein include an infusion cannula 40 that is shorter than the sensor member 30. In any of those examples, the difference in length between the infusion cannula 40 and the sensor member 30 may be selected to provide a desired separation distance between one or more of the sensor electrodes on the sensor member and the outlet of the infusion cannula.

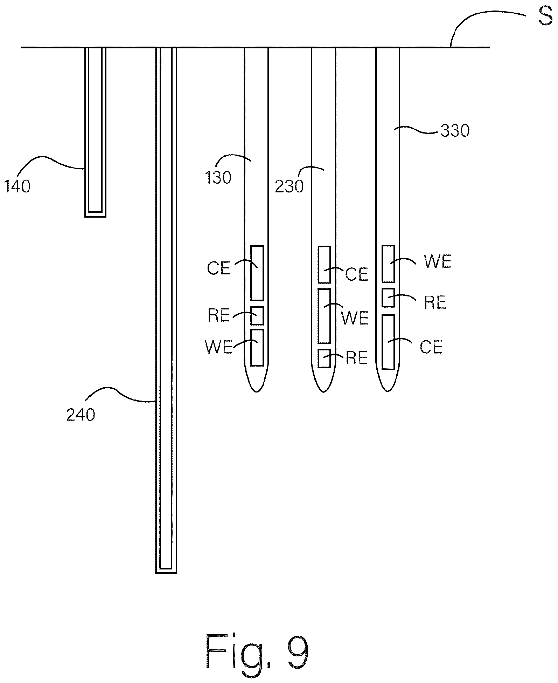

In any of the examples described herein with regard to FIGS. 2-8, the arrangement of the sensor electrodes may be configured to enhance the separation (or provide a desired separation) between a working electrode (WE) on the sensor member and the outlet of the infusion cannula. For example, the diagram in FIG. 9 shows relative lengths of example infusion cannulas and sensor members, including an infusion cannula 140 having a length that is shorter than the length of the sensor member 130, an infusion cannula 240 having a length that is greater than the length of the sensor member 130. The diagram in FIG. 9 also shows three examples of a sensor members 130, 230 and 330, of equal lengths, where each sensor member has an electrode arrangement including a working electrode (WE), a reference electrode (RE) and a control electrode (CE), as described herein. However, the locations of the respective electrodes in the electrode arrangements differs as between the example sensor members 130, 230 and 330. The lengths of the infusion cannulas and the sensor probes are represented in FIG. 9, as the length from the epidermal surface S to the distal ends of the cannulas and probes, when in an inserted state. Each infusion cannula 140 and 240 may correspond to an example of the infusion cannulas 40 discussed herein. Each sensor member 130, 230 and 330 may correspond to an example of the sensor member 30 discussed herein.

To enhance the separation distance between a working electrode (WE) on the sensor member and the outlet of the infusion cannula, a sensor member 130 or a sensor member 230 in which the working electrode (WE) is located closer to the distal end 130a than one or both of the other electrodes, can be employed with an infusion cannula 140 that is shorter than the length of the sensor member. Alternatively, to enhance that separation distance, a sensor member 330 in which the working electrode (WE) is located furthest from the distal end 330a than the other electrodes, can be employed with an infusion cannula 240 that is longer than the length of the sensor member.

In particular examples, the first and second subcutaneous members 30 and 40 (or 130, 140, 230, 240 and 330) are generally flexible along their respective lengths, for example, to flex with movement of adj acent tissue when in an inserted state. In those examples, separate, rigid inserter needles may be used to facilitate inserting the first and the second subcutaneous member 30 and 40 (or 130, 140, 230, 240 and 330) through the patient's skin, to a subcutaneous operating position. In those examples, once the inserter needles have assisted in the insertion of the first and the second subcutaneous member, the inserter needles may be withdrawn from the patient, leaving the first and second subcutaneous members in the inserted state.

As discussed herein with reference to FIGS. 2-4, certain examples include first and the second subcutaneous members 30 and 40 arranged adjacent, but spaced from each other by a distance d and may employ two separate inserter needles that may be extended through the channels 22a and 22b in the base 20, to facilitate insertion of the first and the second subcutaneous member 30 and 40, respectively. In other examples as described herein with reference to FIGS. 5-15, the first and the second subcutaneous members 30 and 40 (or 130, 140, 230, 240 or 330) are arranged to be inserted together, in a single insertion location, such that the first and the second subcutaneous members may be arranged on or within the same inserter needle.

In certain examples, the first and the second subcutaneous members 30 and 40 are arranged within a channel of a single hollow inserter needle, before and during insertion. The first and the second subcutaneous members 30 and 40 may be retained in the needle channel during subcutaneous insertion of the inserter needle. Once inserted to a desired depth, the inserter needle may be axially withdrawn relative to the first and the second subcutaneous members 30 and 40, by sliding the inserter needle along the axial length of the first and the second subcutaneous members, leaving the first and the second subcutaneous members in an inserted state.

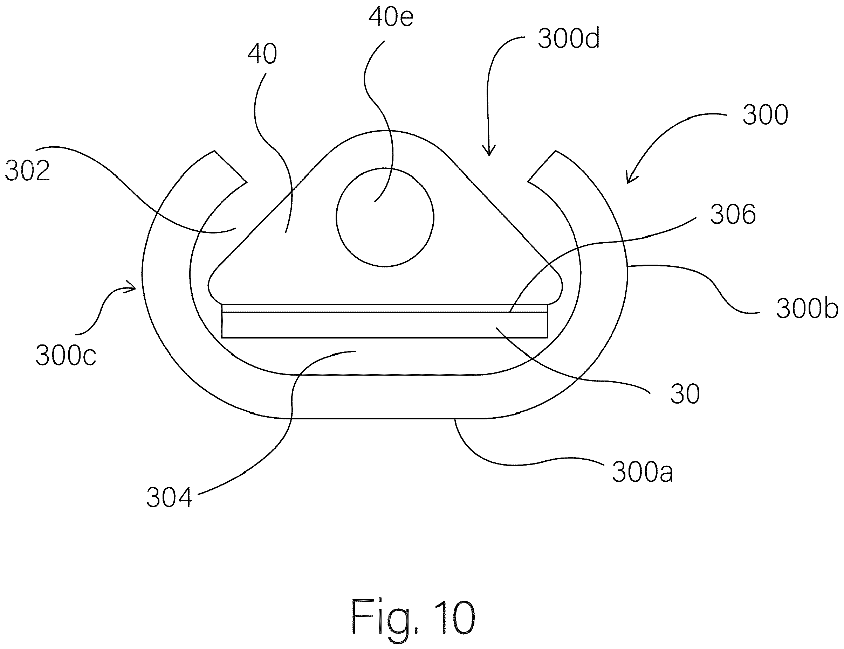

In some examples, the hollow inserter needle may have a slot along its longitudinal dimension, opening to the hollow channel. In other examples, the hollow inserter needle need not include a slot. Examples of various configurations of first and the second subcutaneous members 30 and 40 configured to be disposed within the channel of a slotted, hollow inserter needle are shown in FIGS. 10-15. In those examples, the first and the second subcutaneous members 30 and 40 may correspond to any of the examples of those components shown in Figs. 5-8, or any of the examples 130, 140, 230, 240 or 330 in FIG. 9.

In the example in FIG. 10, an inserter needle 300 is shown in cross-section view (along a cross-section plane that is perpendicular to the longitudinal or axial dimension of the needle). The needle 300 includes a needle shaft (shown in cross-section) made of a generally rigid material such as, but not limited to stainless steel or another metal, ceramic, plastic, composite material, or combinations thereof. The needle shaft is sufficiently rigid along its length dimension to retain its shape without bending or collapsing during insertion of the needle through an epidermal surface S.

The shaft of the inserter needle 300 has a hollow interior or channel 302 extending along the axial length dimension of the inserter needle 300. The first and the second subcutaneous members 30 and 40 are configured to be located within the channel 302 and extend along at least a portion of the axial length of the needle shaft. A distal end portion of the length of the needle shaft may extend beyond the distal ends of the first and the second subcutaneous members 30 and 40, and may include a sharp or tapered tip, to reduce discomfort during insertion of the inserter needle 300. The proximal end of the needle shaft may be connected to a hub or handle, or to a retraction mechanism (not shown) to facilitate retraction and removal of the inserter needle 300 from the first and the second subcutaneous members 30 and 40, after insertion of the inserter needle 300 (with the first and the second subcutaneous members 30 and 40 in the channel 302) to a suitable subcutaneous insertion depth. Accordingly, the inserter needle 300 may be used to insert and place the first and the second subcutaneous members 30 and 40, subcutaneously, with a single needle insertion.

In the example shown in FIG. 10, the shaft of the inserter needle 300 has a cross-section shape that is not perfectly round. Instead, the shape resembles a rounded-tubular shape that is squashed or flattened to some extent, to form a relatively flattened (increased radius) curvature or flat side 300a (shown on the bottom side of the inserter needle 300 in FIG. 10), between two sharper (reduced radius) curved sides 300b and 300c. The curvature of the sides 300b and 300c may increase or flatten out towards a slot 300d (shown as a gap in the cross-section view of FIG. 10). The slot 300d extends along the axial length dimension of the inserter needle 300 and is open to the hollow interior channel of the inserter needle 300.

The relatively flattened peripheral shape of the inserter needle 300 can provide a channel having a similar shape, and a cross-section shape and size suitable for accommodating the first and the second subcutaneous members 30 and 40, simultaneously. In addition, the relatively flattened shape can be beneficial for examples in which the first subcutaneous member 30 includes a sensor substrate (or other sensor element) having a generally flat, ribbon-like shape (and a generally rectangular cross-section shape in a plane perpendicular to the longitudinal axis of the needle 300). In those examples, the sensor substrate (or other sensor element) of the first subcutaneous member 30 may be arranged on the side of the second subcutaneous member 40 that faces the relatively flattened (increased radius) curvature or flat side 300a of the inserter needle 300. As shown in FIG. 10, the relatively flattened (increased radius) curvature or flat side 300a of the inserter needle 300 provides a relatively wide portion of the channel 302 (near the bottom of the channel 302 in FIG. 10) that can accommodate the generally flat, ribbon-like shape of the sensor element.

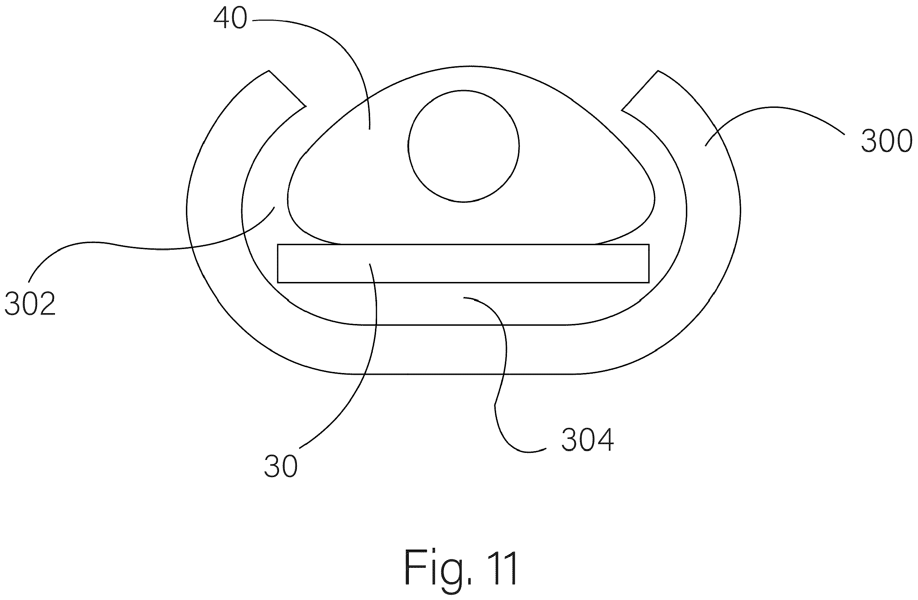

In the example shown in FIG. 10, the second subcutaneous member 40 is an infusion cannula that has a longitudinal axial dimension extending along at least a portion of the length dimension of the channel 302, and a cross-section shape that resembles a "D" shape or a triangle with rounded corners and either flat or curved sides. In FIG. 11, another example of an infusion cannula 40 having a cross-section shape that resembles a "D" shape or a triangle with rounded corners is shown. One side of the infusion cannula 40 (e.g., the downward-facing side in FIG. 10) has a flattened (increased radius or flat) surface that may be abutted against or affixed to the first subcutaneous member 30 (e.g., the sensor element). In other examples, the infusion cannula 40 may have other suitable cross-section shapes.

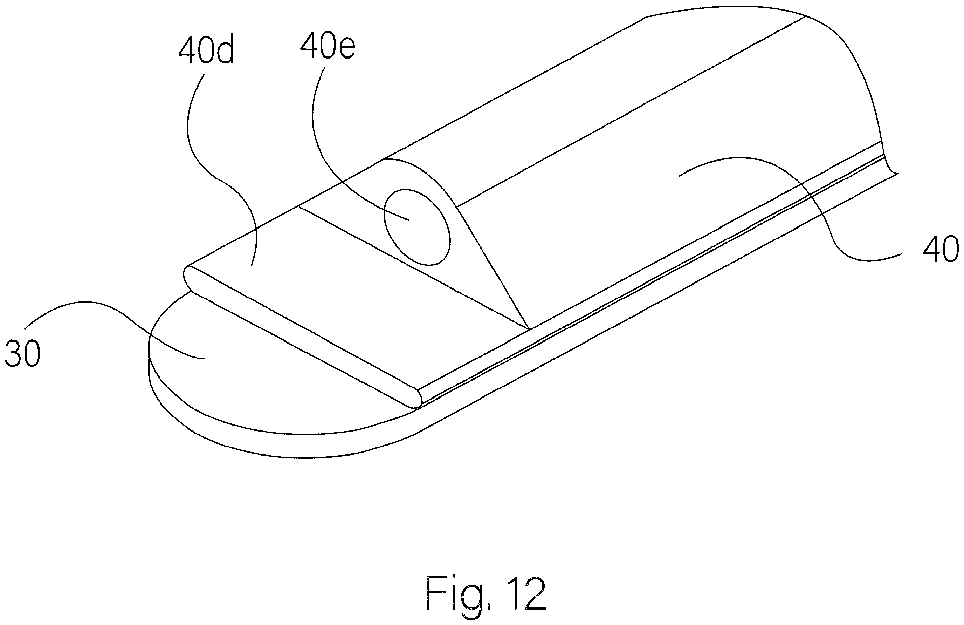

In some examples, the infusion cannula 40 and the sensor element 30 are arranged adjacent each other and are adhered to each other by one or more suitable mechanisms such as, but not limited to an epoxy or other adhesive material layer 306 located between the infusion cannula 40 and the sensor element 30, heat staking, other mechanical connection or the like. In certain examples, as shown in FIG. 12, the infusion cannula 40 may include a flange or extension 40d for facilitating heat staking, where the flange or extension 40d may be heat staked to a surface of the sensor element, such as, but not limited to, the surface of the sensor element facing opposite to the surface on which the sensor electrodes are located. The flattened (increased radius or flat) surface side of the infusion cannula 40 (e.g., the downward-facing side in FIGS. 10 and 11) can provide an increased surface area for adhering to the sensor element 30, to facilitate or improve the adherence of the infusion cannula 40 and the sensor element 30. In other examples, the infusion cannula 40 and the sensor element 30 are arranged adjacent each other, but are not adhered to each other.

The cross-section shape of the infusion cannula 40 (or of the combined infusion cannula 40 and the sensor element 30) may have a width in all dimensions that is greater than the width of the gap defined by the slot 300d in the needle 300. Accordingly, once placed in the channel 302 of inserter needle 300, the infusion cannula 40 (or the combined infusion cannula 40 and the sensor element 30) cannot exit the channel 302, through the slot 300d and, thus, may be retained in the channel 302, until the needle 300 is axially withdrawn relative to the infusion cannula 40.

The infusion cannula 40 in each of FIGS. 10 and 11 has a fluid flow lumen 40e, through which infusion media may flow, after the infusion cannula 40 is subcutaneously inserted in a patient. In certain examples, a portion of the infusion cannula 40 (e.g., the apex of the generally triangular cross-section of the infusion cannula 40) extends at least partially into the gap formed by the slot 300d in the shaft of the needle 300. By allowing a portion of the infusion cannula 40 to extend into that gap, the cross-section size of the infusion cannula 40 can be increased and, thus, the cross-section size or diameter of the fluid flow lumen 40e may be increased (as compared to an infusion cannula that does not extend into that gap). Accordingly, the slotted needle 300 can accommodate an infusion cannula 40 having a relatively large fluid flow lumen 40e, for an increased or improved infusion delivery rate.

The combined infusion cannula 40 and the sensor element 30 in FIGS. 10 and 11 may have a cross-section shape and size that is smaller than the cross-section shape and size of the channel 302, such that the combined infusion cannula 40 and the sensor element 30 may be arranged in the channel 302 without contacting one, two or all side surfaces of the channel. In particular examples, the combined infusion cannula 40 and the sensor element 30 may be arranged such that the sensor element 30 may be spaced from the inner surface of the shaft of the needle 300 (along the relatively flattened or increased radius side 300a) by a gap 304. By providing a space or gap 304 between the sensor element 30 and the inner surface of the needle shaft, the likelihood of the sensor element 30 adhering to the needle shaft can be reduced, e.g., during withdrawal of the needle shaft from the combined sensor element 30 and infusion cannula 40.

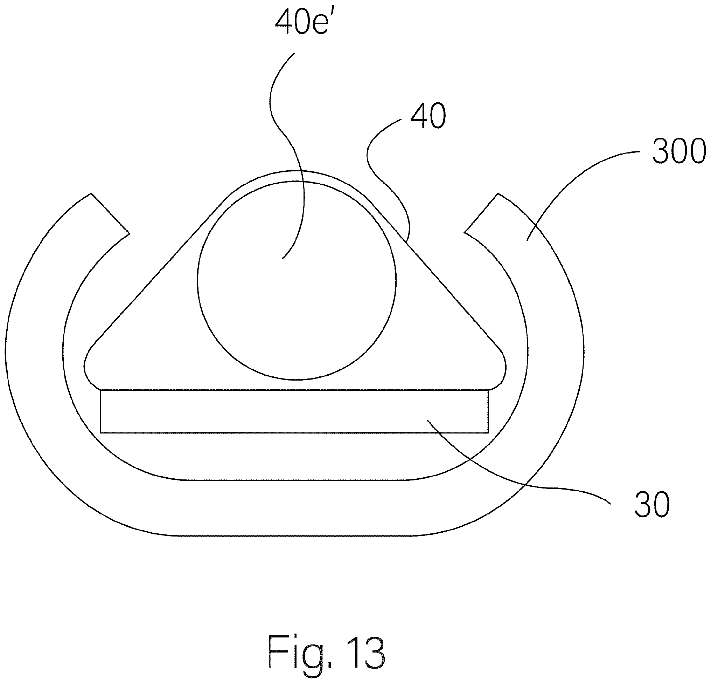

The example in FIG. 13 includes an inserter needle 300, a sensor element 30 and an infusion cannula 40, corresponding to those described above with regard to the example in FIG. 10. However, the infusion cannula 40 in FIG. 13 has a fluid flow lumen 40e' that is larger in diameter than the fluid flow lumen 40e in FIG. 10, for allowing a higher flow rate of infusion media relative to the infusion cannula 40 in FIG. 10. Thus, the generally triangular cross-section shape of the infusion cannula 40 can slide within the channel 302 of the relatively flattened peripheral shape of the inserter needle 300, and may also accommodate a flow lumen 40e, 40e' or a flow channel of any other suitable diameter, based on a desired flow rate or other parameter.

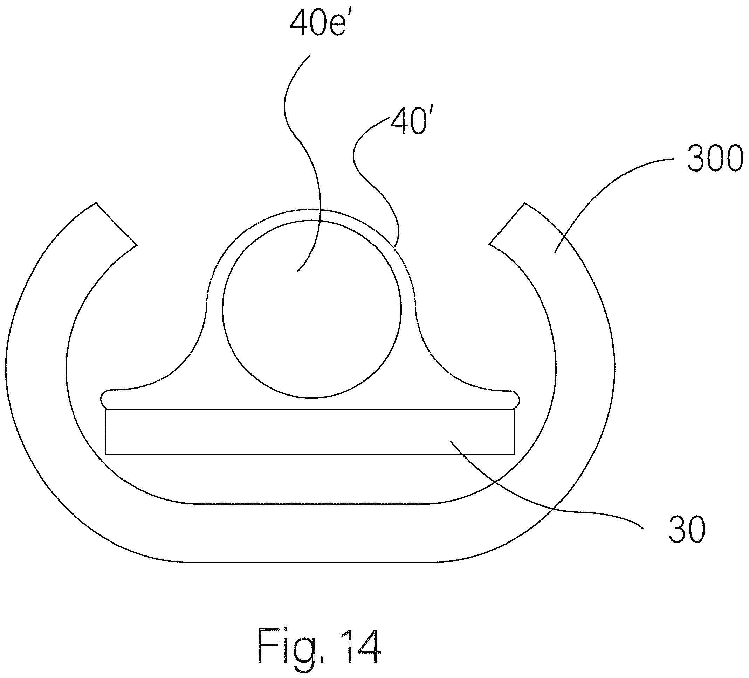

In other examples, such as but not limited to FIG. 14, an infusion cannula 40' may have another suitable shape that can be retained and slide within the channel 302 of the inserter needle 300 without exiting through the gap formed by the slot 300d in the shaft of the inserter needle 300. In the example in FIG. 14, the inserter needle 300, the sensor member 30 and the infusion cannula 40' correspond to the inserter needle 300, the sensor member 30 and the infusion cannula 40 of any of FIGS. 10-13, except that the outer peripheral shape of the infusion cannula 40' is different than the outer peripheral shape of the infusion cannula 40. Similar to the examples in FIGS. 10-13, the cross-section shape of the infusion cannula 40' includes a region or area in which a fluid flow channel 40e' is located. Also similar to the examples in FIGS. 10-123, the cross-section shape of the infusion cannula 40' includes a generally flat or reduced radius surface side (the side facing downward in FIG. 14) for contacting or adhering to the sensor member 30. The example in FIG. 14 differs from the D-shaped or triangular cross-section examples of FIGS. 10-13, in that the outer peripheral shape of the infusion cannula 40' defines curves on each of two sides of the apex, that are directed opposite to the direction of the curve of the apex. The cross-section shape of the infusion cannula 40' in FIG. 14, thus, can be said to resemble a grandfather clock shape.

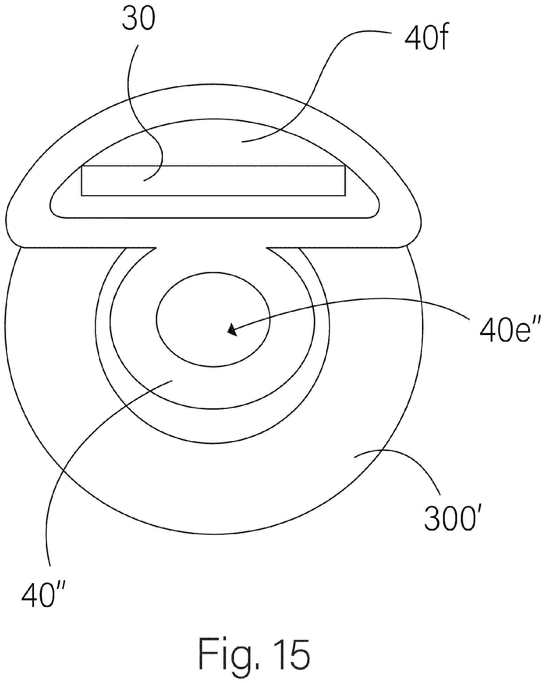

In other examples, such as but not limited to FIG. 15, an infusion cannula 40" may have yet another suitable shape that can be retained and slide within the channel 302 of the inserter needle 300, where the fluid flow lumen 40e" of the infusion cannula 40" is located inside of the needle channel 302, while the sensor element is held within a sensor lumen 40f outside of the needle channel 302.

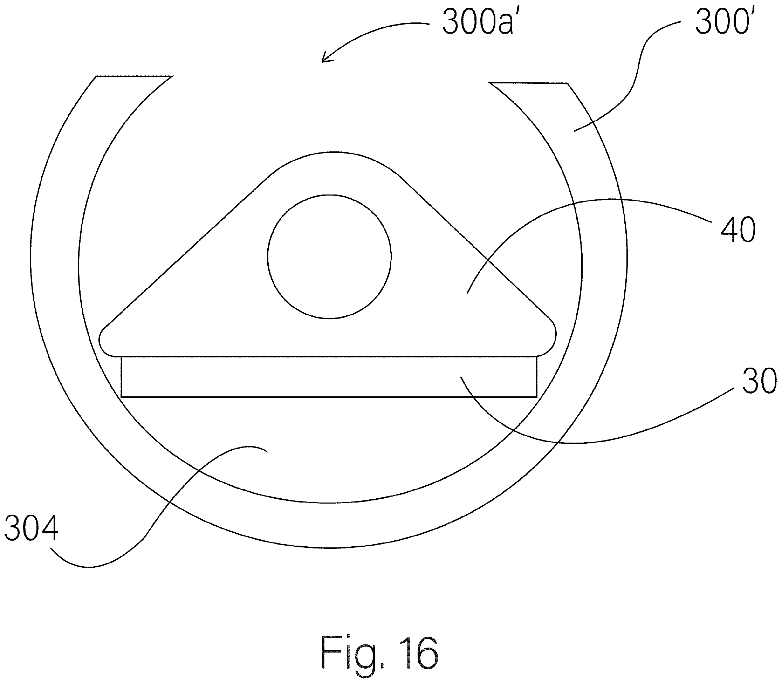

While various examples are described herein as including or operating with an inserter needle 300 having a relatively flattened peripheral shape, other embodiments of any of the examples describe herein may include or be employed with an inserter needle having another suitable shape such as, but not limited to a rounded shape of a typical hypodermic needle. For example, a cross-section view showing a sensor member 30 and an infusion cannula 40 as described above, inside of a slotted inserter needle 300' is shown in FIG. 16, where the slotted inserter needle 300' has a generally circular outer peripheral shape. An axial slot in the needle 300' defines a gap 300a' in the cross-section view.

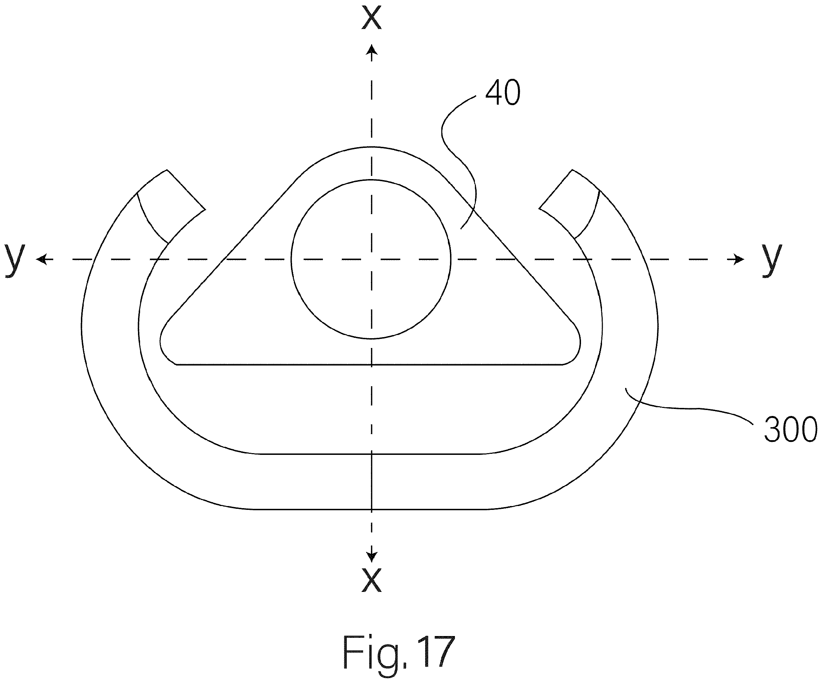

While various examples are described herein as including or operating with a sensor element 30 abutted against or affixed to a flattened (increased radius or flat) surface of the infusion cannula 40, 40', other embodiments of those examples may omit the sensor element 30. For example, as shown in FIG. 17, the subcutaneous member 40 is an infusion cannula as described and shown in FIG. 10 and has a lengthwise dimension extending into and out of the plane of the page (along a z axis). However, in the example in FIG. 17, the flattened (or increased radius or flat) surface of the infusion cannula 40 (the downward-facing surface in FIG. 17) is not abutted against or affixed to a sensor element. In the example in FIG. 17, the infusion cannula 40 may be received within the slotted inserter needle 300 or other rigid inserter needle, and may be inserted through a patient's skin as described with regard to the slotted inserter needle 300 in FIG. 10.

As described above, the D-shaped or generally triangular shaped outer cross-section of the infusion cannula 40 can provide advantages described above with regard to providing a flattened or reduced radius surface to abut against (or affix to) a sensor member 30. However the D-shaped or generally triangular shaped outer cross-section of the infusion cannula 40 can provide other advantages, as well, including but not limited to a resistance to kinking or crimping.

Infusion cannulas can be prone to bending in a patient's subcutaneous tissue, during or after insertion, which can cause kinking or crimping of the cannula lumen and obstruction of fluid flow through the cannula lumen. However, a cannula 40 having a D-shaped or generally triangular shaped outer cross-section configuration can inhibit or reduce kinking of the cannula and avoid or reduce obstruction of fluid flow, when the cannula is bent (particularly along the longest flattened (increased radius or flat) surface, e.g., the downward-facing surface in FIG. 17.

The D-shape or generally triangular shape of the cross-section of the cannula 40 strengthens the resistance to kinking and increases the buckling strength of the cannula 40, relative to a round cross-section cannula with the same inner diameter. The cannula 40 having a D-shaped or generally triangular shaped outer cross-section configuration has a smaller critical kinking radius and, thus, can bend further without kinking, relative to a round cross-section cannula with the same inner diameter, when evaluated via the Brazier effect.

The cannula 40 having a D-shaped or generally triangular shaped outer cross-section exhibits greater resistance to bending in the y-axis direction than in the y-axis direction of FIG.

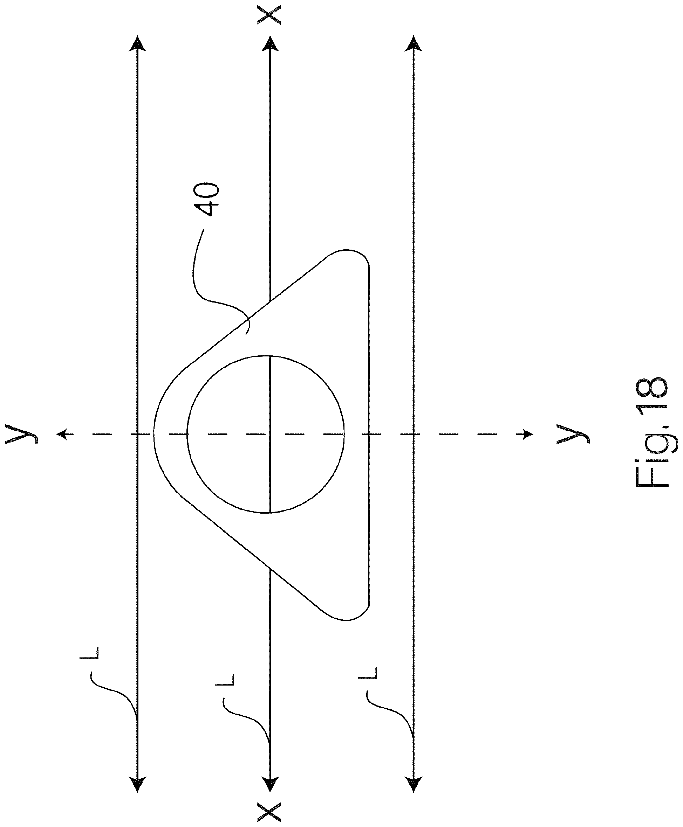

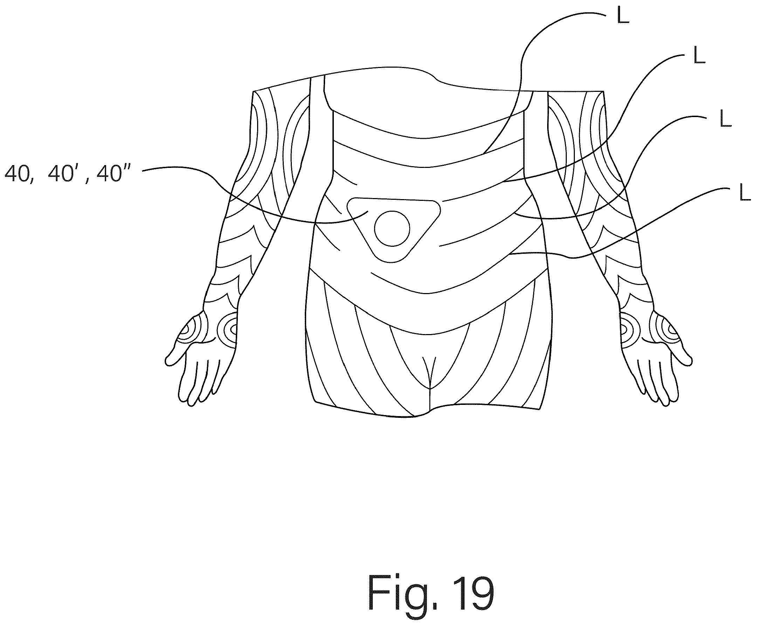

17. Accordingly, in particular examples, the cannula 40 may be configured to be inserted in a patient, with the longest flattened (increased radius or flat) surface, e.g., the downward-facing surface in FIG. 17 oriented generally parallel to the Langer lines L of skin tension of the patient, as represented in FIGS. 18 and 19. The Langer lines L (also called cleavage lines) are parallel to the natural orientation of collagen fibers in the dermis, and underlying muscle fibers of the patient.

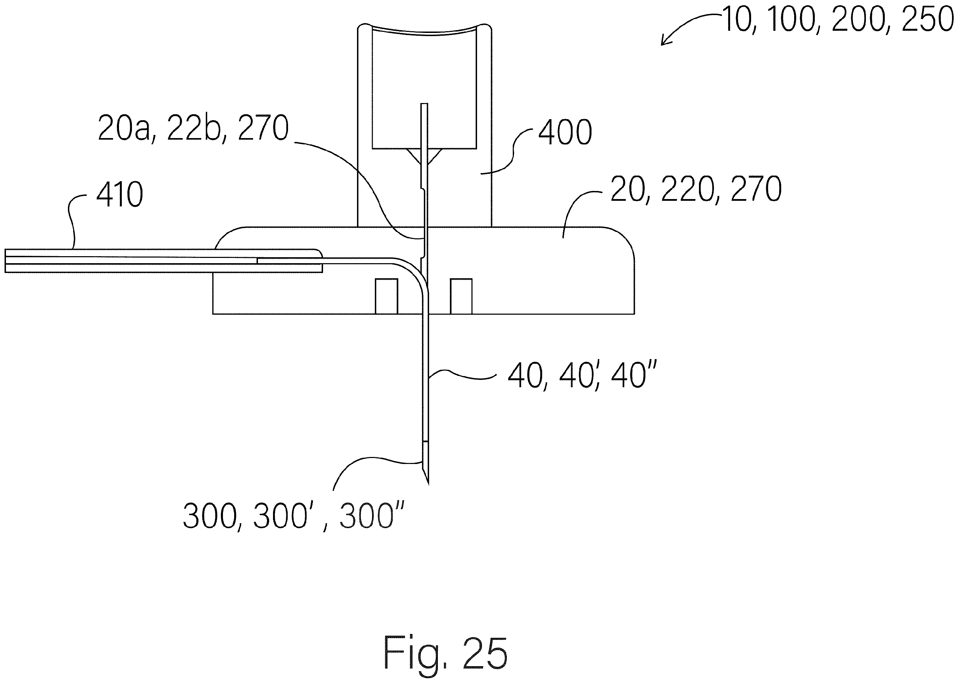

In particular examples, the cannula 40 (or 40' or 40") may be included in a medical device 10, 100, 200, or 300, having a base 20, 220, or 270 from which the cannula extends, as described herein. In certain examples as represented in FIG. 25, an insertion needle 300 or 300' may be held by a needle hub 400 and extended from a surface (the downward facing surface in FIG. 25) of the needle hub 400. The needle hub 400 may be configured to engage with the base 20, 220 or 270, with the insertion needle 300, 300' or 300" extended through the base, by passing the insertion needle 300, 300' or 300" into a needle port and through the channel 22a, 22b or 272. A portion of the insertion needle 300, 300' or 300" extends out from one surface of the base (the downward facing surface in FIG. 25).

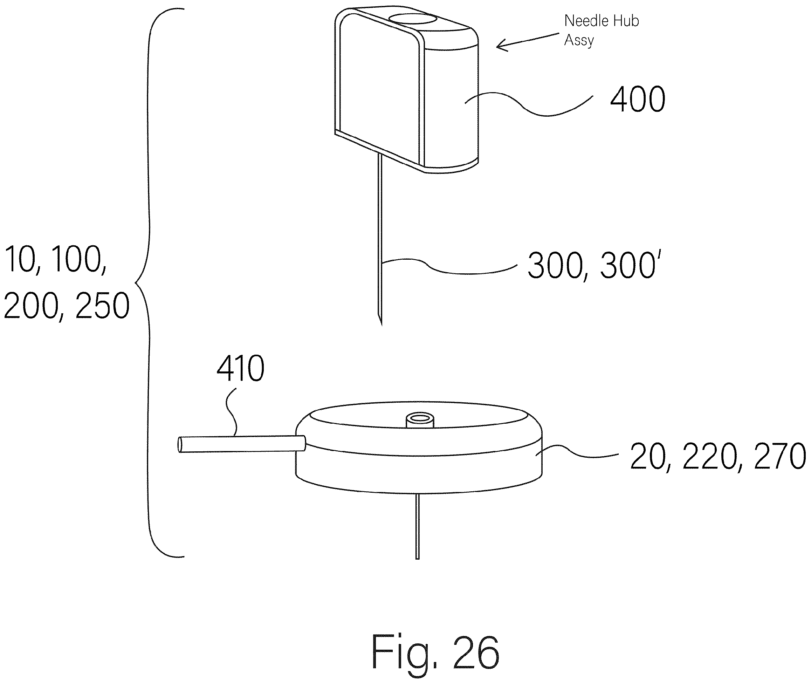

The cannula 40, 40' or 40" has one end connected to an infusion media supply tubing 410 and extends through the slot in the insertion needle 300 or 300' and into the channel of the insertion needle 300 or 300'. The cannula 40, 40' or 40" extends along a portion of the length of insertion needle 300 or 300' that is external to the base 20, 220, or 270, as shown in FIG. 25. In that arrangement, the insertion needle 300 or 300' may assist with the insertion of the cannula 40, 40' or 40" through a patient's skin. Once the cannula 40, 40' or 40" is inserted into the patient, the needle hub (and the insertion needle) may be withdrawn from the base 20, 220, or 270, (as shown in FIG. 26), leaving the cannula in an inserted state.

In particular examples, the cannula 40 (or 40' or 40") may be arranged in a predefined orientation relative to the base 20, 220 or 270 of the medical device 10, 100, 200, 300, such that the user may be instructed to orient the medical device housing in a particular orientation relative to the patient's body (such as shown in FIG. 19) to automatically align the longest flattened (increased radius or flat) surface of the infusion cannula parallel with the Langer lines of the patient. In some examples, the base of the medical device 10, 100, 200, 300 may be configured to be placed on (or adhered to) a patient's skin, and may have a shape or indicia markings (or both) to assist the user with orienting the medical device base in a desired orientation, to result in proper alignment of the cannula 40, 40', 40" relative to the patient's Langer lines.

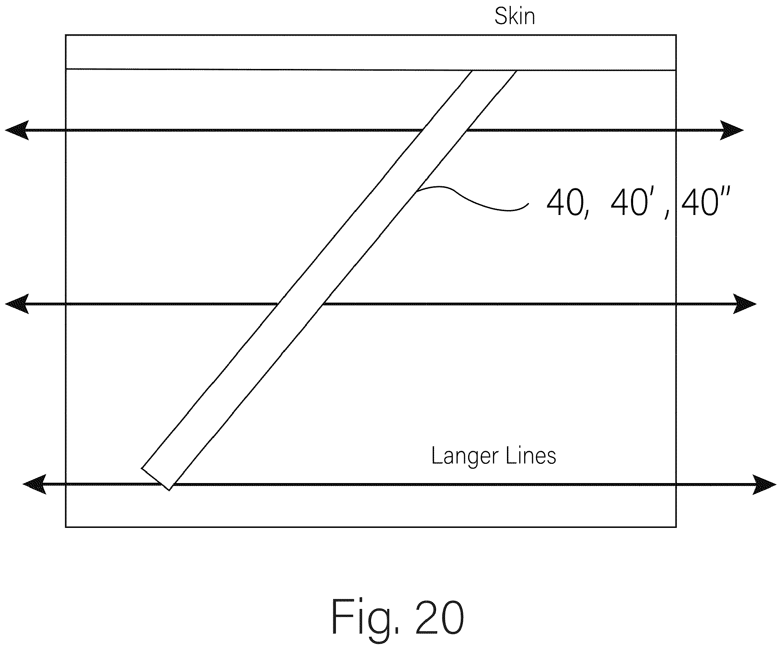

In certain examples, the cannula 40, 40', 40" may be inserted generally parallel to the Langer lines and at an oblique angle relative to the plane of a patient's skin or of the base of a medical device adhered to the patient's skin, as shown in FIG. 20. The oblique angle may be, but is not limited to, an angle in the range greater than or equal to 30 degrees and less than 90 degrees. In other examples, the cannula 40, 40', 40" may be inserted at a 90 degree angle relative to the plane of a patient's skin or of the base of a medical device adhered to the patient's skin.

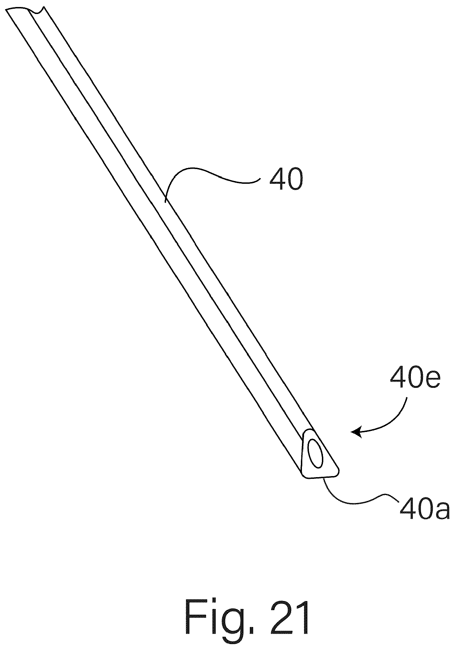

In any of the examples describe herein, the infusion cannula 40, 40', 40" may be provided with a tapered or sharpened tip on its distal end 40a, as shown in FIG. 21. The taper defines an angle relative to the cross-section plane that is perpendicular to the lengthwise axis of the cannula 40, 40', 40". In the example in FIG. 21, the longest flattened (increased radius or flat) surface, e.g., the downward-facing surface in FIGS. 17 and 21, defines the tip of the tapered distal end 40a.

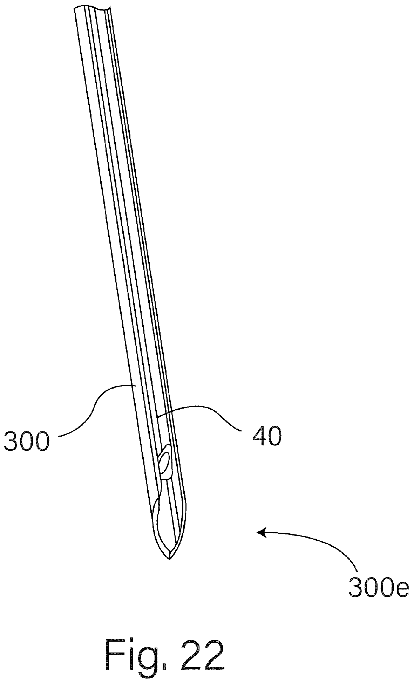

Also in any of the examples described herein, a slotted inserter needle 300 (or 300') may be provided with a tapered or sharpened tip on its distal end 300e, as shown in FIG. 22. The taper defines an angle relative to the cross-section plane that is perpendicular to the lengthwise axis of the inserter needle 300, 300'. In the example in FIG. 22, the side surface 300a, e.g., the downward-facing surface in FIGS. 17 and 22, defines the tip of the tapered distal end 300e. The tapered or sharpened infusion cannula 40 or inserter needle 300, 300' (or both) can reduce insertion trauma and simplify the process of inserting the inserter needle 300 through a patient's skin.

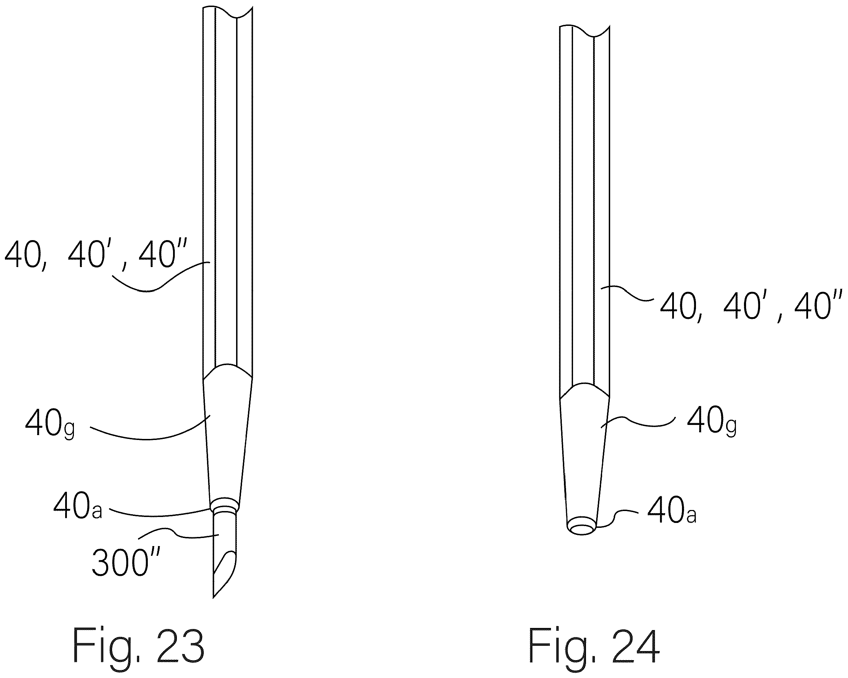

While various examples are described herein as including or operating with a slotted inserter needle 300 (or 300') that has an inner channel in which the infusion cannula 40, 40', 40" (with or without a sensor member 30) is received for insertion, other examples may employ an insertion needle that is not slotted. In yet other examples, an insertion needle 300" may be configured to be received within the lumen of the infusion cannula 40, 40', 40" during insertion, as shown in FIG. 23. The insertion needle 300" in FIG. 23 may be a hollow needle as discussed with regard to insertion needles 300 and 300', or may be a solid (not hollow) insertion needle. In certain examples, a distal end portion 40g of the infusion cannula 40, 40', 40" may be tapered to a smaller outer diameter toward its distal end 40a, towards the smaller outer diameter of the insertion needle 300". Accordingly, the tapered distal end portion 40g may be configured to create a gradual transition between the outer diameter of the insertion needle 300" and the outer diameter of the infusion cannula 40, 40', 40". An infusion cannula 40, 40', 40" having a tapered distal end portion 40g is shown in FIG. 24, without the insertion needle 300".

In any of the examples describe herein, the inserter needle 300 (or 300' or 300"), or other inserter needles for the sensor element 30 described herein, may include a coating or layer of lubricious material to inhibit or reduce sticking of the sensor member 30 (or the infusion cannula 40) to an inner channel surface of the inserter needle. The lubricious material may inhibit sticking and facilitate withdrawing of the inserter needle after insertion of the sensor element, without pulling out the sensor element (or the infusion cannula) with the inserter needle. In certain examples, the inserter needle is dip coated in a 1% Silicone in Hexane solution. Before coating with silicone, the inserter needle is placed in a 0₂ plasma chamber, and the silicone is cured at 25 degrees Celsius and 60% relative humidity after dipping. In other examples, other suitable materials and processes may be used for lubricious coatings or layers including, but are not limited to Teflon, or the like.

In those or other examples, the infusion cannula 40, 40', 40" may include a coating or layer of material to facilitate adhesion of the sensor element 30 to the infusion cannula. In those examples, the adhesion of the sensor element 30 to the infusion cannula can help inhibit the sensor element 30 from being pulled out of an inserted state, as the inserter needle (e.g., 300 or 300') is withdrawn. In those examples, the material to facilitate adhesion may include, but is not limited to a silicon-based polymer, polyurethane, polyethylene, or the like.

In any of the examples described herein, the sensor element 30 may be connected for electrical communication with sensor electronics 50 as discussed above. In particular examples, the sensor electronics and the sensor element 30 may be configured or calibrated (or both) to reduce or minimize interference effects described above. In such examples, the sensor electronics may be configured with one or more processors that operate under an algorithm configured to cancel or counter-act electrical effects of infusion media on the sensor element.

Medical devices 10, 100, 200, 250 as described herein (e.g., with reference to Figs. 2, 3, 4 or 5) may be made according to any suitable manufacturing methods including, but not limited to methods as described herein. In particular examples, the base 20, 220, 270 having one or more channels (e.g., channels 22a, 22b or 272) may be made by molding, machining, or assembly of parts to provide a base structure as described herein. The sensor element 30 may be secured to the base by adhesive material, molding, heat staking, or other mechanical connection. Electronics 50 may be secured to or in the base and electrically connected to the sensor element 30. The septum 60 may be secured to each channel in the base by adhesive material, molding, heat staking, or other mechanical connection. The infusion cannula 40, 40' or 40" may be made by any suitable process including, but not limited to extrusion, machining, molding, micro-molding, or the like. The infusion cannula 40, 40' or 40" may be secured to the base, in fluid flow communication with the channel having the septum 60, by adhesive material, molding, heat staking, or other mechanical connection. An inserter needle 300, 300' is inserted through the septum 60 and through the channel in the base, and out from the bottom side of the base. In some examples, the inserter needle 300, 300' has an inner channel 302 in which the infusion cannula and the sensor element are received, when the inserter needle 300, 300' is inserted through the base. In those examples, the infusion cannula 40, 40' or 40" may extend through the channel in the base and is connected to the septum 60 and, thus, to the base. Alternatively, the base may include a further septum (not shown) through which the infusion cannula 40, 40', 40" and the sensor element 30 extend, for coupling the infusion cannula 40, 40', 40" to the body of the base, and through which the inserter needle 300, 300' extends before and during insertion of the infusion cannula and is withdrawn after insertion.

Once the infusion cannula is received in and extended through the channel of the inserter needle 300, 300' (or the inserter needle 300" is extended through the infusion cannula), the medical device 10, 100, 200, 300 may be readied for installation on a patient. To install the medical device, a backing or release material layer (not shown) may be peeled off of the adhesive material layer on the base 20, 220, 270, to expose the adhesive material on base. Then the base may be placed against the patient's skin at a desired infusion site, such that the inserter needle punctures the patient's skin and is inserted (with the sensor element and the infusion cannula) to an insertion depth, where the adhesive material on the base is in contact with the patient's skin, and adheres the base to the patient. Placement of the base on the patient's skin may be carried out manually, or with an inserter tool. Once the inserter needle has been fully inserted and the base is adhered to the patient's skin, the inserter needle may be withdrawn from the base, through a channel in the base. The inserter needle may be withdrawn manually, or with the aid of an inserter tool. Once the inserter needle has been withdrawn, a connector hub 90 (FIG. 2) or other external infusion media source may be connected in fluid flow communication with the infusion cannula.

While various exemplary embodiments have been presented in the foregoing detailed description, it should be appreciated that a vast number of variations exist. It should also be appreciated that the exemplary embodiment or embodiments described herein are not intended to limit the scope, applicability, or configuration of the claimed subject matter in any way. Rather, the foregoing detailed description will provide those skilled in the art with a convenient road map for implementing the described embodiment or embodiments. It should be understood that various changes can be made in the function and arrangement of elements without departing from the scope defined by the claims, which includes known equivalents and foreseeable equivalents at the time of filing this patent application.

Further disclosed herein is the subject-matter of the following clauses:
1. A medical device comprising:
   a base having a first surface configured to be secured to a patient's skin;
   a first insertable member secured to the base and having a length portion extending from the first surface of the base to a distal end of the first insertable member, for insertion through the patient's skin at an insertion site when the first surface of the base is secured to the patient's skin;
   a second insertable member configured to be secured to the base and having a length portion extending from the first surface of the base to a distal end of the second insertable member, for insertion through the patient's skin at the insertion site when the first surface of the base is secured to the patient's skin;
   wherein the first insertable member includes a sensor member for sensing a biological analyte corresponding to a biological condition;
   wherein the second insertable member includes an infusion cannula for infusing an infusion media; and
   wherein the distal end of the first insertable member and the distal end of the second insertable member are spaced apart by a first distance of at least 5 mm, for reducing interference of the infusion media from the infusion cannula with an operation of the sensor member.
2. The medical device of clause 1, wherein the first insertable member and the second insertable member are spaced apart from each other by a second distance of at least along a plane of the first surface of the base, for insertion in separate, spaced insertion locations, where the second distance is less than the first distance.
3. The medical device of clause 1 or 2, wherein the first insertable member and the second insertable member are arranged adjacent each other for insertion together in a single insertion location.
4. The medical device of clause 3 or of any of the clauses 1 to 3, wherein the length portions of the first insertable member and the second insertable members are attached to each other.
5. The medical device of clause 1 or of any of the clauses 1 to 4, wherein the sensor member has a first length extending from the first surface of the base to the distal end of the sensor member, and the infusion cannula has a second length extending from the first surface of the base to the distal end of the infusion cannula, and wherein the first length is different than the second length.
6. The medical device of clause 5 or of any of the clauses 1 to 5, wherein the sensor member and the infusion cannula are spaced apart from each other along a plane of the first surface of the base by less than the first distance.
7. The medical device of clause 5 or of any of the clauses 1 to 6, wherein the first length is greater than the second length, the sensor member has a first surface that faces toward the infusion cannula and that is connected to the infusion cannula, and the sensor member has at least one electrode on the first surface of the sensor member for interfacing with biological fluid or tissue after the sensor member is inserted at the insertion site.
8. The medical device of clause 5 or of any of the clauses 1 to 7, wherein the sensor member has a first surface that faces toward the infusion cannula and a second surface that faces in an opposite direction as the first surface of the sensor member, and the sensor member has at least one electrode on the second surface of the sensor member for interfacing with biological fluid or tissue after the sensor member is inserted at the insertion site.
9. The medical device of clause 1 or of any of the clauses 1 to 8, wherein the infusion cannula has fluid flow lumen along an axial length dimension of the infusion cannula, and at least one side wall opening in fluid flow communication with the lumen for expelling infusion media through a side wall of the infusion cannula, and wherein each side wall opening is provided on a side of the infusion cannula that faces away from the sensor member.
10. The medical device of clause 1 or of any of the clauses 1 to 9, wherein the length portion of at least one of the first and second insertable members extends from the first surface of the base at an oblique angle relative to the first surface of the base.
11. The medical device of clause 1 or of any of the clauses 1 to 10, wherein the length portion of each of the first and second insertable members extends from the first surface of the base at an oblique angle relative to the first surface of the base.
12. The medical device of clause 1 or of any of the clauses 1 to 11, further comprising an inserter needle having a hollow channel along a lengthwise axial dimension of the inserter needle, wherein the sensor member and the infusion cannula are arranged adjacent each other in the hollow channel of the inserter needle, for insertion together at a single insertion location.
13. The medical device of clause 12 or of any of the clauses 1 to 12, wherein the infusion cannula has a first side that has a reduced radius or flat surface facing the sensor member, and wherein the infusion cannula is attached to the sensor member along at least a portion of the first side of the infusion cannula by one or more of an adhesive or heat staking.
14. The medical device of clause 12 or of any of the clauses 1 to 13, wherein the inserter needle has a slot along its lengthwise axial dimension, and wherein a portion of the infusion cannula extends at least partially into the slot.
15. The medical device of clause 12 or of any of the clauses 1 to 14, wherein the inserter needle is configured to slide in a direction of its lengthwise axial dimension relative to the sensor member and to the infusion cannula, for selectively withdrawing the inserter needle relative to the sensor member and the infusion cannula, and wherein at least one of the inserter needle or the infusion cannula includes a coating or layer for reducing friction between the inserter needle and one or both of the infusion cannula and the sensor member.
16. The medical device of clause 12 or of any of the clauses 1 to 15, wherein the base has a channel through which the inserter needle extends for insertion of the sensor member and the infusion cannula.
17. The medical device of clause 16 or of any of the clauses 1 to 16, wherein the infusion cannula is connected in fluid flow communication with the channel in the base, and wherein the base includes at least one septum located adjacent or within the channel in the base, through which the inserter needle extends for insertion of the sensor member and the infusion cannula.
18. The medical device of clause 17 or of any of the clauses 1 to 17, wherein the at least one septum provides a port on the base for receiving a needle or rigid cannula of an infusion media source that provides infusion media to the infusion cannula.
19. A system including a medical device of clause 18 or of any of the clauses 1 to 18, and further comprising:
   at least one infusion media source including a syringe or other fluid dispenser having a needle through which fluid is dispensed; or
   a connector hub having a rigid cannula configured to extend through the septum to connect a fluid channel in the connector hub in fluid flow communication with the port on the base, the fluid channel in the connector hub being connected in fluid flow communication, through a tubing, to a pump and a reservoir of infusion media.
20. The medical device of clause 1 or of any of the clauses 1 to 18, wherein the biological analyte is at least one of glucose, ketone or lactose.
21. An infusion cannula comprising a tubular member having a lengthwise dimension and a lumen extending along the lengthwise dimension, the tubular member having a cross-section shape in a plane perpendicular to the lengthwise dimension, where the cross-section shape comprises a generally triangular shape or D-shape, defining a side surface that is flat or has a greater radius than each of the other side surfaces of the cross-section shape.
22. The infusion cannula of clause 21, wherein the tubular member has a distal end that is tapered.
23. A medical device comprising:
   a housing configured to be placed on or adhered to a patient's skin;
   an infusion cannula supported by the housing to extend through the patient's skin when the housing is placed on or adhered to the patient's skin, the infusion cannula comprising a tubular member having a lengthwise dimension and a lumen extending along the lengthwise dimension, the tubular member having a cross-section shape in a plane perpendicular to the lengthwise dimension, where the cross-section shape comprises a generally triangular shape or D-shape, defining a side surface that is flat or has a greater radius than each of the other side surfaces of the cross-section shape;
   wherein the housing has at least one shape or indicia marking that identifies a desired orientation at which the housing may be placed or adhered to the patient's skin; and
   wherein the infusion cannula is oriented such that a longest side of the generally triangular shape or D-shape cross-section of the infusion cannula is about parallel with the Langer lines of the patient.

## Claims

1. A medical device comprising:
a base having a first surface configured to be secured to a patient's skin;
a first insertable member secured to the base and having a length portion extending from the first surface of the base to a distal end of the first insertable member, for insertion through the patient's skin at an insertion site when the first surface of the base is secured to the patient's skin;
a second insertable member configured to be secured to the base and having a length portion extending from the first surface of the base to a distal end of the second insertable member, for insertion through the patient's skin at the insertion site when the first surface of the base is secured to the patient's skin;
wherein the first insertable member includes a sensor member for sensing a biological analyte corresponding to a biological condition;
wherein the second insertable member includes an infusion cannula for infusing an infusion media; and
wherein the distal end of the first insertable member and the distal end of the second insertable member are spaced apart by a first distance of at least 5 mm, for reducing interference of the infusion media from the infusion cannula with an operation of the sensor member.

2. The medical device of claim 1, wherein the first insertable member and the second insertable member are spaced apart from each other by a second distance of at least along a plane of the first surface of the base, for insertion in separate, spaced insertion locations, where the second distance is less than the first distance, or
wherein the first insertable member and the second insertable member are arranged adjacent each other for insertion together in a single insertion location, particularly
wherein the length portions of the first insertable member and the second insertable members are attached to each other.

3. The medical device of claim 1 or 2, wherein the sensor member has a first length extending from the first surface of the base to the distal end of the sensor member, and the infusion cannula has a second length extending from the first surface of the base to the distal end of the infusion cannula, and wherein the first length is different than the second length, and/or
wherein the biological analyte is at least one of glucose, ketone or lactose.

4. The medical device of any of the claims 1 to 3, wherein the sensor member and the infusion cannula are spaced apart from each other along a plane of the first surface of the base by less than the first distance.

5. The medical device of claim 3 or 4, wherein the first length is greater than the second length, the sensor member has a first surface that faces toward the infusion cannula and that is connected to the infusion cannula, and the sensor member has at least one electrode on the first surface of the sensor member for interfacing with biological fluid or tissue after the sensor member is inserted at the insertion site.

6. The medical device of any of the claims 1 to 5, wherein the sensor member has a first surface that faces toward the infusion cannula and a second surface that faces in an opposite direction as the first surface of the sensor member, and the sensor member has at least one electrode on the second surface of the sensor member for interfacing with biological fluid or tissue after the sensor member is inserted at the insertion site.

7. The medical device of any of the claims 1 to 6, wherein the infusion cannula has fluid flow lumen along an axial length dimension of the infusion cannula, and at least one side wall opening in fluid flow communication with the lumen for expelling infusion media through a side wall of the infusion cannula, and wherein each side wall opening is provided on a side of the infusion cannula that faces away from the sensor member, and/or
wherein the length portion of at least one of the first and second insertable members extends from the first surface of the base at an oblique angle relative to the first surface of the base, and/or
wherein the length portion of each of the first and second insertable members extends from the first surface of the base at an oblique angle relative to the first surface of the base.

8. The medical device of any of the claims 1 to 7, further comprising an inserter needle having a hollow channel along a lengthwise axial dimension of the inserter needle, wherein the sensor member and the infusion cannula are arranged adjacent each other in the hollow channel of the inserter needle, for insertion together at a single insertion location.

9. The medical device of any of the claims 1 to 8, wherein the infusion cannula has a first side that has a reduced radius or flat surface facing the sensor member, and wherein the infusion cannula is attached to the sensor member along at least a portion of the first side of the infusion cannula by one or more of an adhesive or heat staking.

10. The medical device of claim 8 or 9, wherein the inserter needle has a slot along its lengthwise axial dimension, and wherein a portion of the infusion cannula extends at least partially into the slot, and/or
wherein the inserter needle is configured to slide in a direction of its lengthwise axial dimension relative to the sensor member and to the infusion cannula, for selectively withdrawing the inserter needle relative to the sensor member and the infusion cannula, and wherein at least one of the inserter needle or the infusion cannula includes a coating or layer for reducing friction between the inserter needle and one or both of the infusion cannula and the sensor member.

11. The medical device of any of the claims 8 to 10, wherein the base has a channel through which the inserter needle extends for insertion of the sensor member and the infusion cannula, particularly
wherein the infusion cannula is connected in fluid flow communication with the channel in the base, and wherein the base includes at least one septum located adjacent or within the channel in the base, through which the inserter needle extends for insertion of the sensor member and the infusion cannula.

12. The medical device of claim 11, wherein the at least one septum provides a port on the base for receiving a needle or rigid cannula of an infusion media source that provides infusion media to the infusion cannula.

13. A system including a medical device of claim 12, and further comprising:
at least one infusion media source including a syringe or other fluid dispenser having a needle through which fluid is dispensed; or
a connector hub having a rigid cannula configured to extend through the septum to connect a fluid channel in the connector hub in fluid flow communication with the port on the base, the fluid channel in the connector hub being connected in fluid flow communication, through a tubing, to a pump and a reservoir of infusion media.

14. An infusion cannula comprising a tubular member having a lengthwise dimension and a lumen extending along the lengthwise dimension, the tubular member having a cross-section shape in a plane perpendicular to the lengthwise dimension, where the cross-section shape comprises a generally triangular shape or D-shape, defining a side surface that is flat or has a greater radius than each of the other side surfaces of the cross-section shape, particularly wherein the tubular member has a distal end that is tapered.

15. A medical device comprising:
a housing configured to be placed on or adhered to a patient's skin;
an infusion cannula supported by the housing to extend through the patient's skin when the housing is placed on or adhered to the patient's skin, the infusion cannula comprising a tubular member having a lengthwise dimension and a lumen extending along the lengthwise dimension, the tubular member having a cross-section shape in a plane perpendicular to the lengthwise dimension, where the cross-section shape comprises a generally triangular shape or D-shape, defining a side surface that is flat or has a greater radius than each of the other side surfaces of the cross-section shape;
wherein the housing has at least one shape or indicia marking that identifies a desired orientation at which the housing may be placed or adhered to the patient's skin; and
wherein the infusion cannula is oriented such that a longest side of the generally triangular shape or D-shape cross-section of the infusion cannula is about parallel with the Langer lines of the patient.
